(19) Europäisches Patentamt
European Patent Office
Office européen des brevets

(11) Publication number: **0 435 725 B1**

(12) **EUROPEAN PATENT SPECIFICATION**

(45) Date of publication of patent specification: **01.02.95**

(51) Int. Cl.$^{6}$: **C12N 15/55**, C12N 9/16

(21) Application number: **90403529.2**

(22) Date of filing: **11.12.90**

(54) **DNA having the genetic information of phospholipase D and its use.**

(30) Priority: **15.12.89 JP 325355/89**

(43) Date of publication of application:
**03.07.91 Bulletin 91/27**

(45) Publication of the grant of the patent:
**01.02.95 Bulletin 95/05**

(84) Designated Contracting States:
**DE FR GB IT**

(56) References cited:
**WO-A-89/09818**

**J. BIOCHEM., vol. 85, no. 1, 1979, pages 79-95, S. IMAMURA et al.: "Purification of Streptomyces chromofuscus Phospholipase D by Hydrophobic Affinity Chromatography on Palmitoyl Cellulose"**

**JOURNAL OF BACTERIOLOGY, vol. 170, no. 12, December 1988, pages 5855-5862, Baltimore, US; M. GIVSKOV et al.: "Cloning and Expression in Escherichia coli of the Gene for Extracellular Phospholipase A1 from Serratia liquefaciens"**

(73) Proprietor: **Asahi Kasei Kogyo Kabushiki Kaisha**
**2-6, Dojimahama 1-chome**
**Kita-ku**
**Osaka-shi**
**Osaka 530 (JP)**

(72) Inventor: **Yoshioka, Issei**
**707-1, Ohitocho-Mifuku**
**Tagata-gun,**
**Shizuoka 410-23 (JP)**
Inventor: **Mizoguchi, Junzo**
**357-8, Ohitocho-Mikado**
**Tagata-gun,**
**Shizuoka 410-23 (JP)**
Inventor: **Takahara, Masayasu**
**774-1, Ohitocho-Yoshida**
**Tagata-gun,**
**Shizuoka 410-23 (JP)**
Inventor: **Imamura, Shigeyuki**
**696, Ohitocho-Mifuku**
**Tagata-gun,**
**Shizuoka 410-23 (JP)**
Inventor: **Beppu, Teruhiko**
**1-5-21, Horinouchi**
**Suginami-ku,**
**Tokyo 166 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**WORLD PATENT INDEX, abstract no. 74-31479V (17), 1974, Derwent Publications Ltd., London, GB**

Inventor: **Horinouchi, Sueharu**
**2-406 Nishichiba Shukusha,&**
**1-170, Yayoicho**
**Chiba-shi,**
**Chiba 260 (JP)**

(74) Representative: **Bourgognon, Jean-Marie et al**
**Cabinet Flechner**
**22, Avenue de Friedland**
**F-75008 Paris (FR)**

## Description

The present invention relates to a phospholipase D gene and a process for producing a phospholipase D with use of the gene.

### Background Art

Phospholipase D (Phosphatidylcholine Phosphatidohydrolase; referred to hereinafter as PLD) is an enzyme which acts one, for example, lecithin (phosphatidylcholine) as a phospholipid to decompose the ester bond of the phospholic acid and the choline moiety and to liberate phosphatidic acid and choline. It is known to be present in the leaves of cabbage, spinach, turnip, potato, lettuce or the like, the roots of carrot, sugar beet, beet or the like, the plant tissues of barley buds, cotton seeds, bean sprouts of green peas or the like (see KOSO HANDBOOK, pp. 451 - 452, 1982, Asakura shobo), or in microorganisms such as Streptomyces genus (see Japanese Patent Publication Nos. 52-39918, and 60-4716 (US 4,135,980)) or Nocardiopsis genus (see Japanese Patent Publication No. 63-62195), and the purification of phospholipase D extracted from the culture medium of Streptomyces chromofuscus has also been reported in J. Biochem., 85, 79 - 95 (1979).

Phospholipase D is known to be an enzyme which is utilized for a reagent of determinating blood phopholipids and for the production of derivatives by the hydrolysis of a phospholipid or the base exchange reaction with use of the reaction of the phospholipase (see Japanese Patent Laid-Open Publication Nos. 63-36790 and 61-236793).

As described above, while various phospholipase D producing microorganisms have been reported, phospholipase D produced by these phospholipase D producing microorganisms was different from the other microorganisms in specificity to a substratae, and the amino acid sequences of the phospholipase D remained ambiguous, not to speak of the presumption of the amino acid sequences of the phospholipase D produced from individual phospholipase D producing microorganisms. Under these situations, it has been desired to find a method for efficiently producing a phospholipase D having a high purity and a high quality because of a low production efficiency of phospholipase D obtained particularly by extracting it from the culture medium of S. chromofuscus and purifying it.

Also, the primary structure of the polypeptide which constitutes phospholipase D and the base sequence of DNA which codes for the amino acid sequence of the enzyme have not hitherto been reported.

Therefore, it has been desired to determine the primary structure of the polypeptide which constitutes phospholipase D, to determine the base sequence of DNA which codes for the amino acid sequence of the enzyme and to produce the enzyme by genetic engineering.

Fig. 1 shows the amino acid sequence of the polypeptide which constitutes phospholipase D;

Fig. 2 shows the DNA which codes for the amino acid sequence of the polypeptide which constitutes phospholipase D;

Fig. 3 shows the total base sequence of the DNA of a potrion derived from actinomycetes of plasmid pcPLD1;

Fig. 4 shows the restriction enzyme map of Escherichia coli plasmid pcPLD1;

Fig. 5 shows the restriction enzyme map of actinomycetes plasmid pcPLD1;

Fig. 6 shows the restriction enzyme site of the phospholipase D gene, wherein ▨ represents the signal sequence; and

Fig. 7 shows the construction of a plasmid, wherein ▭ represents the phospholipase D gene.

The present inventors have conducted earnest researches on the aforementioned problems. As a result, they have isolated and purified the gene coding for the amino acid sequence of the polypeptide which constitutes phospholipase D from a microorganism belonging to Streptomyces chromofuscus, determined the primary structure of the polypeptide which constitutes the enzyme and the base sequence of the DNA which codes for the amino acid sequence of the enzyme.

Furthermore, they have established and excellent process for industrially producing the phospholipase D by introducing the phospholipase D gene into an optional vector to derive a transformant from a host microorganism with a host-vector system, culturing the transformant to express the phospholipase D gene information and identifying phospholipase D from the culture. The present invention has been thus completed.

The present invention is defined in the claims.

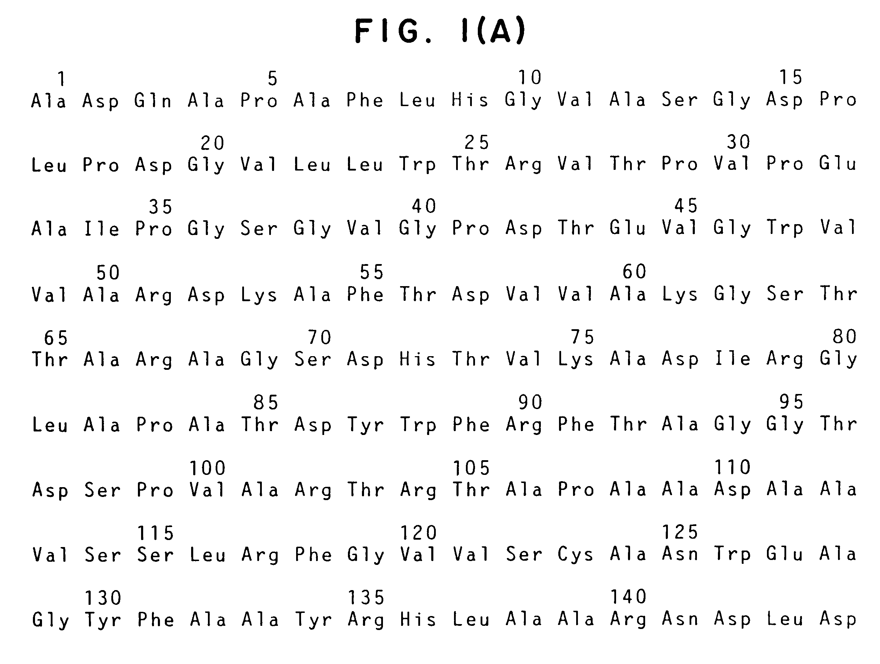

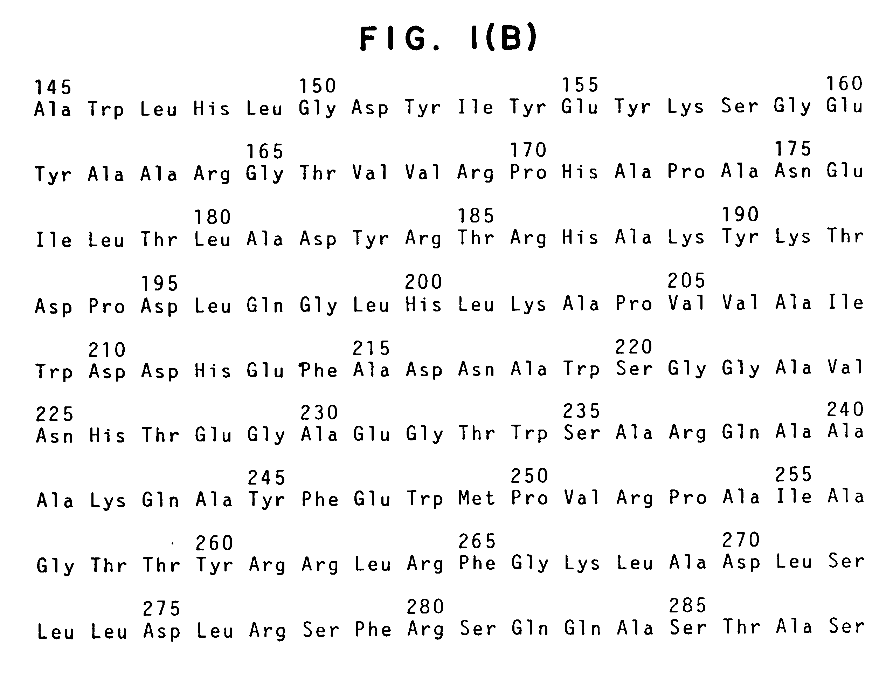

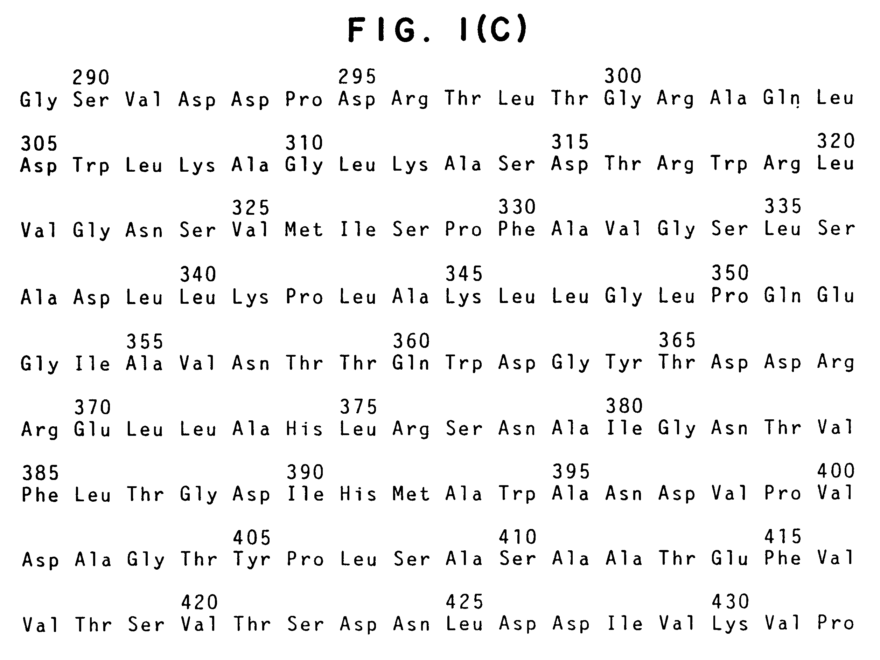

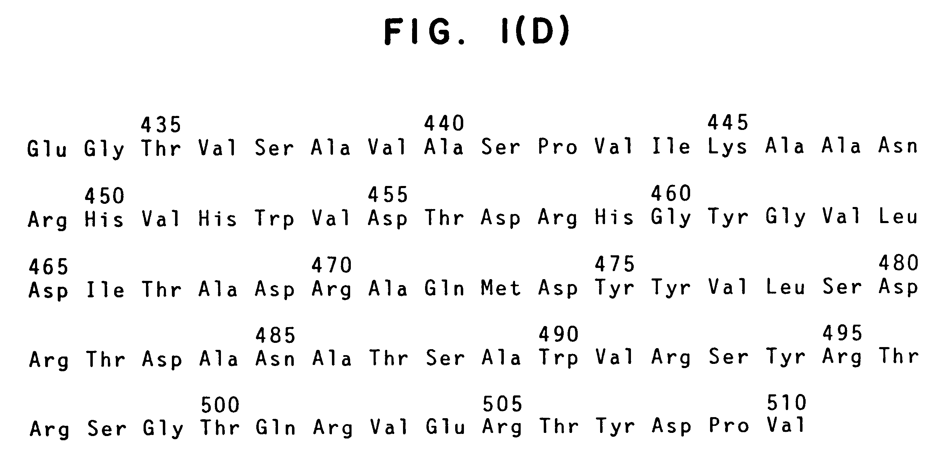

In the DNA according to the present invention which codes for the amino acid sequence represented by Fig. 1, the N-terminal side and the C-terminal side of the amino acid sequence represented by Fig. 1 may contain an amino acid moiety or a polypeptide moiety, and one or more amino acid moieties may be

present upstream to Ala in the N-terminal side. As the amino acid moiety, there are mentioned an initiation codon or a signal peptide. One or more amino acid moieties may be present downstream to Val in the C-terminal side.

Furthermore, the amino acid sequence constituting phospholipase D may be a part of the amino acid sequence represented by Fig. 1 which expresses the same effect as the expression of enzyme activity by a polypeptide comprising the amino acid sequence represented by Fig. 1.

The novel DNA according to the present invention which codes for the amino acid sequence represented by Fig. 1 must only a DNA comprising any one codon among series of codons corresponding to amino acids, respective, in the amino acid sequence including the amino acid moiety or polypeptide moiety in the N-terminal side or the C-terminal side.

Further, the DNA coding for the amino acid sequence which constitutes phospholipase D according to the present invention may be a DNA coding for a part of the amino acid sequence in Fig. 1 which expresses the same effect as the expression of enzyme activity by a polypeptide comprising the amino acid sequence represented by Fig. 1.

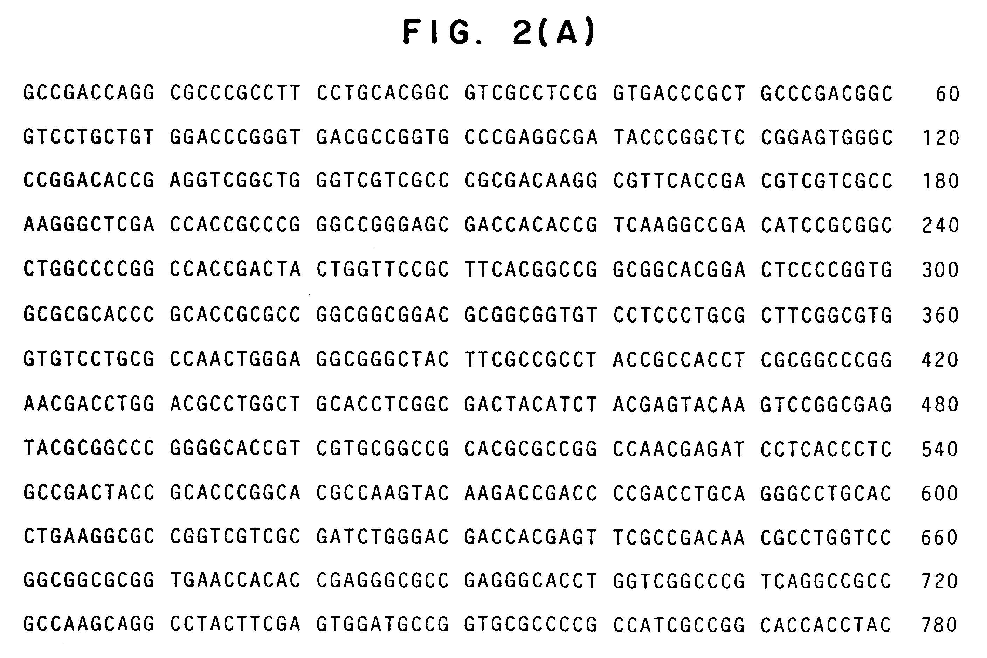

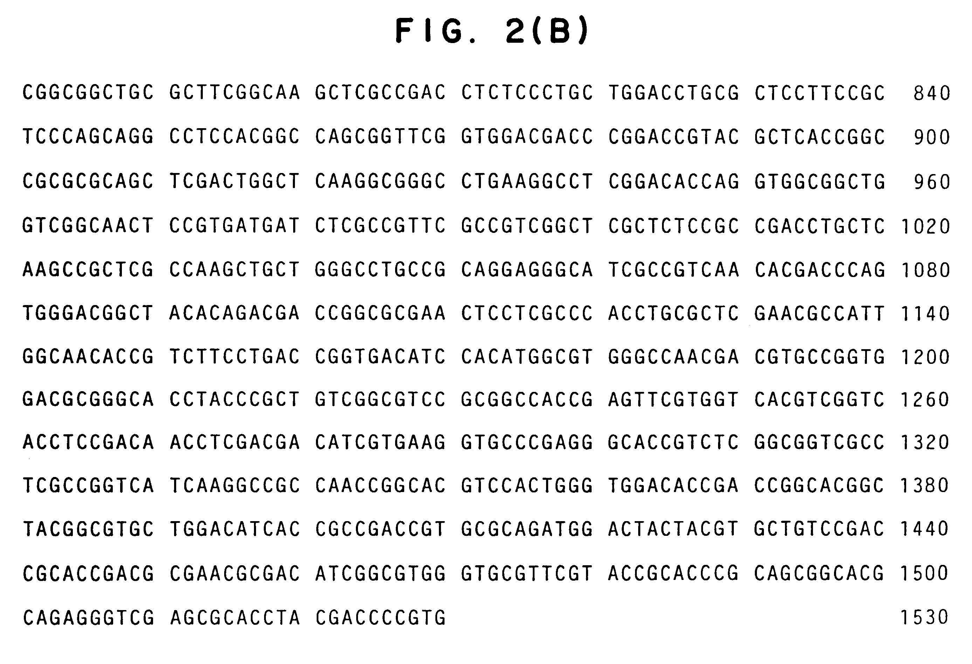

As a typical of the DNA described above, there can be mentioned a DNA containing a base sequence from 5′-terminal side represented by Fig. 2. The DNA may contain one or more codons which code for amino acids upstream to the 5′-terminus and must only be codons other than TAA, TAG and TGA. Further, there can be mentioned DNA which preferably contains ATG, GTG, initiation codons other than the two or codons corresponding to a signal peptide. The DNA may contain downstream to GTG as the 3′-terminus one or more codons which code for amino acids or a translation terminating codon. When the DNA contains one or more codons which code for amino acids in the 3′-terminal side, it preferably contain a translation terminating codon at the 3′-terminus of the codon which codes for the amino acids.

The DNA coding for the amino acid sequence represented by Fig. 1 or the DNA represented by Fig. 2 may be produced, for example, by isolating and purifying the DNA of a microorganism as a donor of a phospholipase D gene which produces phospholipase D from the microorganism, ligating the DNA which has been cleaved with supersonic, a restriction enzyme or the like and an expression vector which has been cleaved into a linear form at the blunt or cohesive end of these DNA to form a cycle, transferring the recombinant DNA vector thus obtained to a replicalbe host microorganism, culturing the recombinant DNA vector retaining microorganism which is obtained by screening with indices of the marker of the vector and the activity of phospholipase D, isolating and purifying the recombinant DNA vector from the cultured microorganism cells and harvesting the DNA as the phospholipase D gene from the recombinant DNA vector.

As the microorganism as the DNA donor, there may be used actinomycetes, preferably S. chromofuscus A-0848 strain (FERM P-3519, BP-361) or its redeposition strain S. chromofuscus A-0848 (FERM BP-2682).

As for the bacteriological properties, culturing means and purification means of the bacterium strain, there is described in Japanese Patent Publication No. 60-4716 (from line 3 from the bottom of column 29 at page 15 to line 4 of column 34 at page 17).

The DNA derived from the microorganism as a donor of gene is harvested in the following procedures.

For instance, the microorgainsm as the donor described above is cultured in a liquid culture medium in a jar fermentor for about 1-3 days, and the culture obtained is centrifuged to collect the microorganism, which is then lysed to prepare a lysate containing the phospholipase D gene. As the method of lysis, the microorganism is treated with a cell wall lytic enzyme such as lysozyme or $\beta$-glucanase, if necessary, in combination with other enzymes such as protease or the like or surfactants such as sodium laurylsulfate or the like. The freezing and shawing method or the French press treatment as the physical destruction methods of the cell wall (see Japanese Patent Laid-Open Publication No. 63 - 185371) may be used in combination with the aforementioned lysis method.

The isolation of purification of the DNA from lysate thus obtained can be carried out in usual manners such as the deproteination treatment by the phenol extraction, the protease treatment, the ribonuclease treatment, the alcohol precipitation or centrifugation in an appropriate combination with each other.

While the microorganismic DNA thus isolated and purified can be cleaved by the treatment, for example, with supersonic or a restriction enzyme, the treatment with a restriction enzyme, particularly a restriction enzyme of the type II such as Sal I, Sac I, Kpn I, Xho I or Mlu I which act on the specific nucleotide sequences is preferred for the easy lygation of the DNA fragment thus obtained and a vector.

As the vector, a vector which has been constructed as a vector for genetic recombination from a phage which is autonomously propagatable in a host microorganism or a plasmid.

As the phage vector, there may be used λgt, λC, λgt or λB when Escherichia coli is used as the host microorganism.

Also, as the plasmid vector, there may be used plasmids such as pBR 322, pBR 325, pACYC 184, pUC 12, pUC 13, pUC 18, pUC 19, pUC 118, pIN I or the like for E. coli. Similarly, there may be used plasmids such as pTUB, pTUB 285 or the like for Bacillus subtilis, a plasmid pAM 82 for Saccharomyces cerivisiae, plasmids such as pSEV 1 (Mol. Gen. Genet., (1987), 210, 468 - 475 (FERM BP-2684)), pIJ 680 or the like for Streptomyces lividans. Furthermore, a shuttle vector which can be autonomously propagated in two or more host microorganisms such as E. coli and S. lividans can be used.

Such vectors are preferably cleaved with the same restriciton enzyme as the one used for the cleavage of the microorganismic DNA as the donor of the phospholipase D gene to give a vector fragments.

The method of ligating the microorganismic DNA fragment and the vector fragment may be any methods in which a well-known DNA ligase is used. For example, the cohesive end of the microorganismic DNA fragment and the cohesive end of the vector fragment are annealed, and a recombinant DNA is next prepared from the microorganismic DNA fragment and the vector fragment by the effect of an appropriate DNA ligase. If necessary, it is also possible to transfer the annealed fragments into the host microorganism and to prepare a recombinant DNA with use of the DNA ligase present in the microorganism.

The host microorganism may be any one in which the recombinant DNA can be stably and autonomously propagated and the character of an exogenous DNA can be expressed. When the host microorganism is an microorganism belonging to E. coli, E. coli DH1, E. coli HB 101, E. coli W 3110, E. coli C 600 or the like can be used. When the host microorganism is an microorganism belonging to S. lividans, S. lividans TK-24 (FERM BP-2685), S. lividans TK-21, S. griseofuscus, S. coelicolor, S. ambofaciens or the like can be used.

As the method for introducing the recombinant DNA into the host microorganism, there may be used a method for introducing a recombinant DNA in the presence of a calcium ion, when the host microorganism is the one belonging to the Escherichia genus. When the host microorganism is the one belonging to the Bacillus genus, there may be used the competent cell method, the introducing method by electro fusion of a ribosome recombinant DNA into a protoplast host cell and the microinjection method.

The presence of the introduction of an objective recombinant DNA into a host microorganism may be carried out by radioactive labelling of the DNA probe of a preliminarily synthesized phospholipase D with $^{32}P$ or the like, colony hybridizing the labelled DNA probe with a preliminarily anticipated gene library and selecting a positive strain as an objective transformant.

The aforementioned gene manipulation has generally a relationship in quantity of about 1-10 $\mu$g of a restriction enzyme, about 300 $\mu$g of a ligase and about 1 - 10 $\mu$g of other enzymes to 0.1 - 10 $\mu$g of a DNA from a donor microorganism and a plasmid DNA.

The microorganism as the transformant thus obtained such as a microorganism belonging to E. coli has an ability of stably producing a large amount of phospholipase D on culturing the microorganism in a nutrient medium.

As an embodiment of the transformant, there is mentioned E. coli DH 1•pcPLD1 (FERM BP-2683) which is obtained by incorporating the DNA represented by Fig. 2 into a plasmid pUC 118 (TAKARA SHUZO K.K.), transfoming into E. coli DH 1 (ATCC 33849) [T. Maniatis et al., Molecular Cloning: Cold Spring Harbor (1982), 504 - 506] as a host microorganism, and selecting a microorganism which produces phospholipase D.

The recombinant DNA thus selected can be easily taken out from the recombinant microorganism retaining the recombinant DNA and introduced again into the other host microorganism.

Furthermore, the DNA which codes for the amino acid sequence of the polypeptide constituting phospholipase D can be cut out from the recombinant DNA with an enzyme such as a restriction enzyme and ligated with the terminus of the other open ring vector which is obtained by the cleavage in the same manner as above to make a recombinant DNA having new characteristics, which is then easily introduced into the other host microorganism.

As an embodiment of the recombinant DNA, there is mentioned S. lividans TK-24•pSEV 1-PLD (FERM BP-2686) which is obtained by incorporating the DNA represented by Fig. 2 into a plasmid pSEV1 (FERM BP-2684), transforming into S. lividans TK-24 (FERM BP-2685) as a host microorganism, and selecting a microorganism which produces phospholipase D.

The phospholipase D of the present invention may be subjected to a well-known gene manipulation to cause the mutation of a peptide. Such mutein DNA indicates an artificial mutant gene which is produced from the phospholipase D of the present invention by some genetic technology. The artificial mutant gnee is obtained by the use of various genetic technologies such as the site specific base transformation method and the substitution of a specific DNA fragment of an objective gene with an artificial mutant DNA fragment. The phospholipase D mutein DNA having extremely excellent properties among artificial mutant genes thus obtained can be finally inserted into a vector to produce a recombinant DNA, which is then introduced into a

host microorganism to produce a phospholipase D mutein.

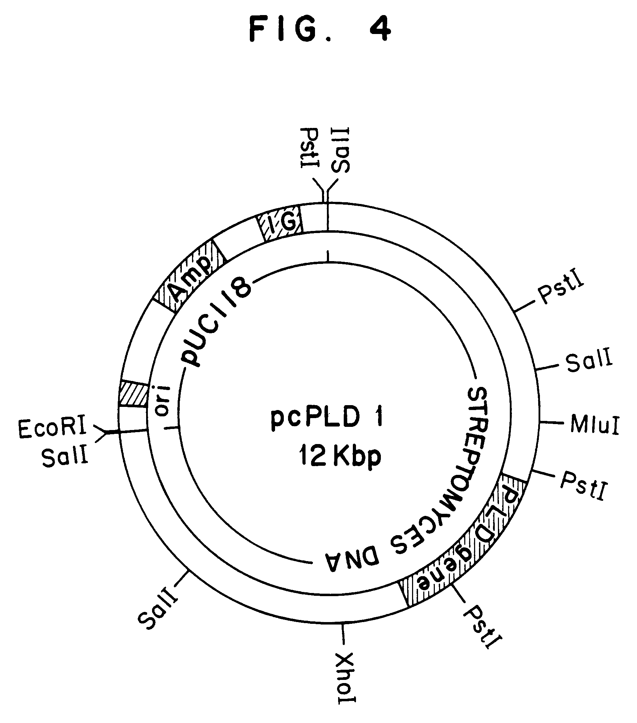

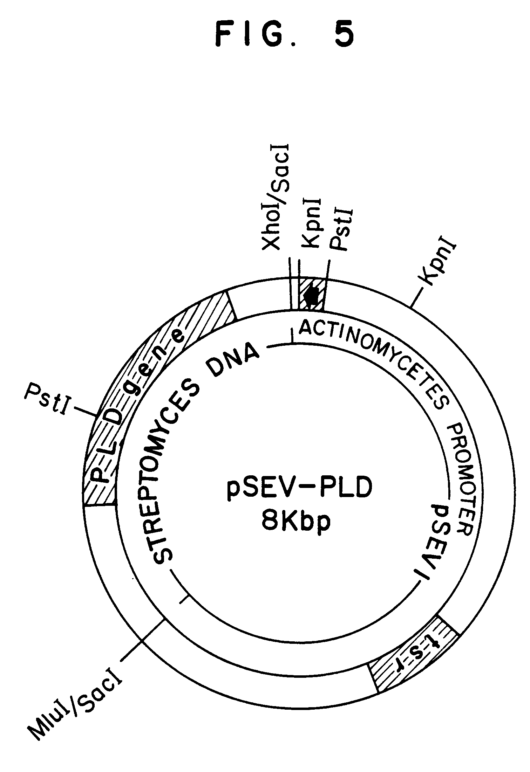

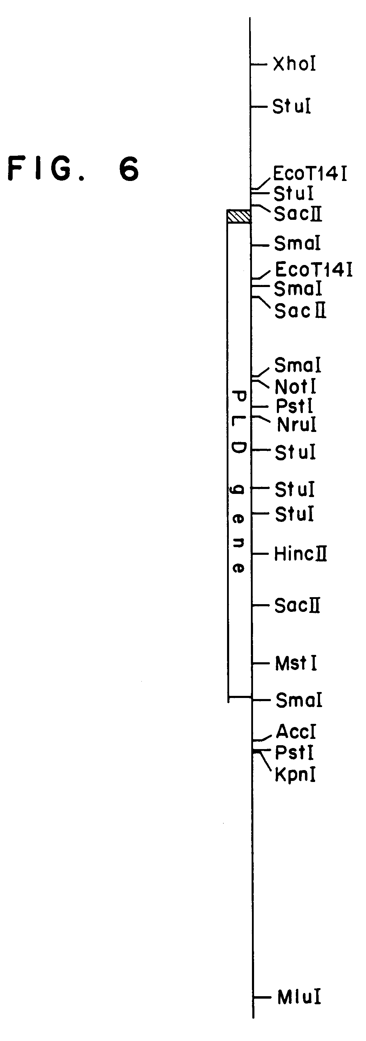

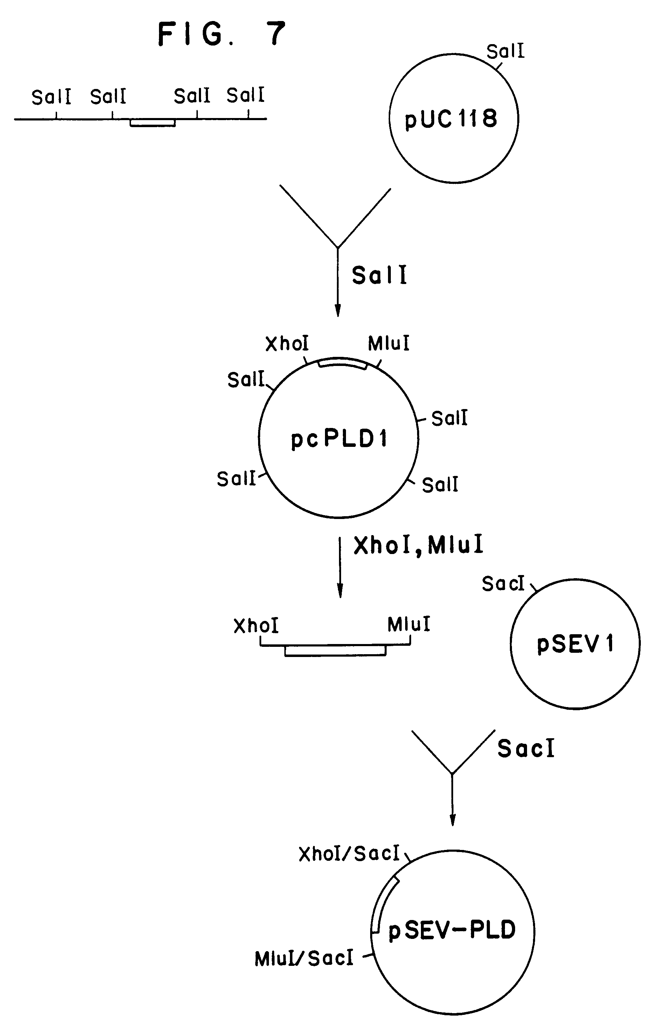

As the embodiments of vectors which contain the DNA coding for phospholipase D, ther are mentioned the plasmid pcPLD1 taken from E. coli DH1•pcPLD 1 and the plasmid pSEV-PLD taken from S. lividans TK-24 (Figs. 4 and 5 show their restriction enzyme maps). Also, the restriction enzyme cleavage map of the PLD gene obtained from the plasmid pcPLD 1 is shown in Fig. 6 (wherein ▨ indicates a signal peptide). The base sequence of the DNA coding for the amino acid sequence of the polypeptide which constitutes the phospholipase D obtained by the aforementioned method was decoded by the dideoxy method disclosed in Science, 214, 1205 - 1210 (1981), and the total amino acid sequence of the polypeptide which constitutes the phospholipase D was estimated from the base sequence. It was also confirmed that as for the polypeptide as the phospholipase D having been cultured and purified by the method described below, the N-terminal amino acid sequecne measured by a liquid phase protein sequencer (Beckman System 890 ME; manufactured by Beckman) was in accordance with a part of the estimated amino acid sequence. The construction of the plasmid pcPLD1 and the plasmid pSEV-PLD are illustrated in Fig. 7 (wherein ▭ indicates the phospholipase D gene).

The production of the phospholipase D with a transformant can be carried out by culturing the transformant in a nutrient culture medium to have the phospholipase D produced in the microorganismic cell or in the culture medium, harvesting the cell by some means such as filtration or centrifugation of the culture obtained after culturing, destroying the cell by a mechanical method or an enzymatic method with lysozyme or the like, adding, if necessary, EDTA and/or an appropriate surfactant to solubilize the phospholipase D, and separating and collecting as an aqueous solution. The aqueous solution of the phospholipase D thus obtained can be, with or without condensation, subjected to ammonium sulfate fractionation, gel permeation, absorption chromatography such as affinity chromatography or the like, or ion exchange chromatography to give a phospholipase D having a high purity.

The culturing mode of a microorganism as a transformant may be selected in consideration of the nutrient physiological properties. While the microorganism is cultured in liquid, submerged culture in a jar fermenter is advantageous to the industrial production of the microorganism. As a nutrient source of the culture medium, a culture medium which is usually used for the culture of microorganisms can be widely used.

It is sufficient that the carbon source is a carbon compound which can be used as a nutrient, and there are used, for example, glucose, saccharose, lactose, maltose, fructose, molasses or the like. It is sufficient that the nitrogen source is a nitrogen compound which can be used as nutrient, and there are used, for example, peptone, broth, yeast extract, casein hydrolysate or the like. There may be also used, if necessary, phosphates, carbonates, sulfates, salts of magnesium, calcium, potassium, iron, manganese, zinc or the like, specific amino acids, specific vitamins, or the like.

The culturing temperature can be varied appropriately within the temperature range that the microorganism grows and produces the phospholipase D, and it is preferably in the range of 20 - 42°C for E. coli and 28 - 30°C for S. lividans. While the culturing time varies depending on the conditions, it is sufficient that the culturing is finished at an appropriate time when the phospholipase D reaches a maximum yield. The culturing time is usually in the range of 12 - 48 hours for E. coli and 48 - 72 hours for S. lividans. The pH of the culture medium can be appropriately varied within the range that a microorganism can grow and produce phospholipase D, and it is preferably in the range of 6.0 - 8.0 for E. coli and 7.0 - 7.5 for S. lividans.

The phospholipase D in the culture can be directly harvested and utilized in the form of a culture medium containing the microorganismic cells. In general, when the phospholipase D is present in a culture medium, it is utilized after separating the phospholipase D containing solution from the microorganismic cells by filtration, centrifugation or the like. When the phospholipase D is present within the microorganismic cell, the culture obtained is subjected to filtration, centrifugation or the like to harvest the microorganismic cell, which is then destroyed by a mechanical method or an enzymatic method with lysozyme or the like. Then, a chelating agent such as EDTA or the like and/or a surfactant are added to the destroyed cell mixture, and the phospholipase D is solubilized, then separated and collected in the form of an aqueous solution.

It is sufficient that the phospholipase D containing solution thus obtained is subjected to, for example, condensation under reduced pressure, membrane condensation, a salting out treatment with ammonium sulfate, sodium sulfate or the like, or a fractional precipitation method with a hydrophilic organic solvent such as methanol, ethanol, acetone or the like to give a precipitate.

Next, the precipitate can be dissolved in water and subjected to dialysis through a semipermeable membrane to remove impurities having a lower molecular weight. The product is further purified by gel filtration with use of an adsorbent or a gel filter medium, absorption chromatography such as affinity

chromatography or the like, ion exchange chromatography or the like, and the phospholipase D containing solution obtained by these purification procedures are subjected to condensation under reduced pressure, lyophilization or the like to give a purified phospholipase D.

As the phospholipase D obtained by the aforementioned producing methods, there is mentioned, for example, a phospholipase D having the following physical properties:

(1) Action

Phospholipase D has an action on the ester bond of the phosphoric acid and a nitrogen containing base of a phospholipid to cleave into a phophorus compound and a corresponding nitrogen containing compound. The action of the phospholipase D on lecithin is represented as follows:

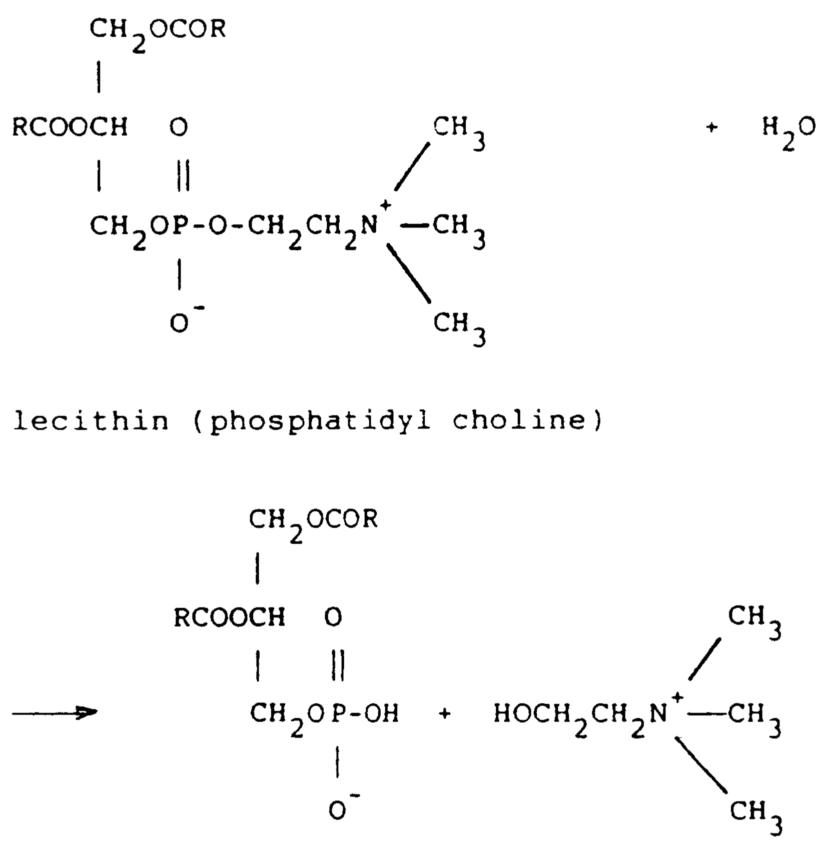

lecithin (phosphatidyl choline)

phosphatidic acid          choline

Furthermore, lecithin is hydrolyzed with phospholipase D, so that it is also possible to produce a nucleic acid type nucleoside-phospholipid complex having a carcinostatic effect by the base-exchange reaction between the substrates of nucleic acid type nucleoside and a phospholipid such as lecithin in the reversible reaction.

(2) Determination of activity

| | |
|---|---|
| 0.2 M Tris-hydrochloric acid buffer (pH 8.0) | 0.1 ml |
| 10 mM lecithin emulsion | 0.1 ml |
| 0.1 M calcium chloride | 0.05 ml |
| 1% Triton X100 | 0.1 ml |
| Water | 0.1 ml |

To the reaction mixture having a composition as above was added 0.05 ml of the enzyme solution. After reaction it 37 ° C for 10 minutes, the mixture was boiled for 5 minutes to stop the reaction. The reaction solution was cooled to 37 ° C. Then, 0.1 ml of 4-aminoantipyrine (3 mg/ml), 0.1 ml of Phenol (2 mg/ml), 0.1 ml of peroxidase (2 U/ml), 0.1 ml of choline oxidase (30 U/ml) and 0.1 ml of water were added to the solution, the reaction was conducted at 37 ° C for 20 minutes before adding 2 ml of 1% Triton X100 to the reaction mixture for measuring the absorbance at 500 nm. It is defined that 1 Unit (U) of phospholipase D has an enzyme activity of producing 1 μmole/min of choline. The enzyme activity is calculated from the following equation:

$$U/ml = \Delta A_{500} \times 0.55$$

wherein $\Delta A_{500}$ denotes the absorbance at 500 nm.

(3) Substrate specificity

The same procedure as described above for the reaction solution for the measurement of the activity was repeated except that emulsions (10 mM, 0.1 ml) of lecithin, lysolecithin and sphingomyelin, respectively, as substrates were used. The amount of choline was determined, and the relative enzyme activities are calculated in terms of the enzyme activity to lysolecithin as 100.

| Substrate | Relative Activity |
|---|---|
| Lecithin | 51% |
| Sphingomyelin | 19% |
| Lysolecithin | 100% |

(4) Stability to pH

pH 5: Acetate buffer
pH 6 - 7: Dimethyl glutarate-sodium hydroxide buffer
pH 8 - 9: Tris-hydrochloric acid buffer
pH 10: Glycine-sodium hydroxide buffer.

After the enzyme solution is incubated in the buffer solutions (10 mM) at 37 ° C for 60 minutes, the activity of the phospholipase D is measured in accordance with the aforementioned method for determining the enzyme activity.

The enzyme is comparatively stable at pH 7 - 9.

(5) Optimal pH

pH 6 - 7.5: Dimethyl glutarate-dosium hydroxide buffer
pH 7 - 9: Tris-hydrochloric acid buffer
pH 9 - 10: Glycine-sodium hydroxide buffer.

The activity of the phospholipase D is measured in accordance with the aforementioned method for determining the enzyme activity. The optimal pH is in the range of about 7.5 - 8.

(6) Thermal stability

After the phospholipase D is incubated at each temperature of 40, 50, 60, 70 and 80 °C for 10 minutes under the condition of 10 mM Tris-hydrochloric acid buffer (pH 8.0) and 1 mg/ml of the concentration of the enzyme solution, the activity of the phospholipase D is measured in accordance with the aforementioned method for determining the enzyme activity. The enzyme is stable at a temperature of up to 70 °C.

(7) Molecular weight and isoelectric point

The phospholipase D has a molecular weight of about 50,000 measured by Sephadex G-150 gel filtration and about 57,000 measured by SDS-polyacrylamide disk electrophoresis.

The phospholipase D has an isoelectric point (IP) of pH 5.0.

The phospholipase D thus obtained is superior to a phospholipase D obtained from the Streptomyces hachijoensis A 1143 strain (Japanese Patent Laid-Open Publication No. 48 - 99386) in the optimum pH, the stability to pH and the thermal stability. As the S. hachijoensis A 1143 strain produces phospholipase C in addition to phospholipase D (Japanese Patent Laid-Open Publication No. 49 - 55893), it has a disadvantage of causing a side reaction due to the contamination of phospholipase C into a final product unless substantially completely purified. On the other hand, a phospholipase D having a high purity is obtained by a easy purification procedure according to the present invention, and thus the phospholipase D can be said to be a particularly useful enzyme.

While the present invention is explained in detail with reference to examples, it should be understood that the invention is not limited thereto.

Example 1

Extraction of DNA from actinomycetes

Into 500 ml of Troptic Soy Broth (30 g/ℓ, manufactured by DIFCO) was inoculated a phospholipase D producing actinomycetes S. chromofuscus A 0848 (FERM BP 2682), and shake culture was conducted at 30 °C for 3 days. The culture solution was centrifuged at 14,000 rpm (25,000 G) for 10 minutes in a high-speed refrigerated centrifuge (HITACHI SCR-20BA Model) to collect the actinomycetes cells. The actinomycetes cells were suspended in 20 ml of TES (50 mM Tris-HCl pH 8.0, 50 mM EDTA pH 8.0, 15% sucrose), and lysozyme (manufactured by SIGMA Co.) was added in such an amount as the final concentration is of 1 mg/ml. Treatment was conducted at 37 °C for 10 minutes to destroy the cell wall. Then, 0.5 ml of 10% SDS was added, and 21 ml of a mixture of phenol and chloroform (1 : 1) was further added before centrifugation at 14,000 rpm (25,000 G) for 15 minutes. The aqueous layer separated was placed in the other vessel, and 42 ml of ethanol was added. Chromosome deposited was collected by entwining it around a glass rod.

The chromosome was dissolved in 20 ml TE (10 mM Tris-HCl pH 8.0, 1 mM EDTA pH 8.0), and 20 ml of a mixed solution of phenol and chloroform (1 : 1) was added before stirring. Then, centrifugation was carried out at 14,000 rpm (25,000 G) for 15 minutes. The aqueous layer separated was placed in the other vessel, and 2 ml of 3 M sodium acetate buffer (pH 5.5) and 50 ml of ethanol were added before stirring. The mixture was cooled at -70 °C for 20 minutes and centrifuged at 3,000 rpm (2,000 G) for 15 minutes, and the chromosome deposited was washed with 75% ethanol and dried to give 550 µg of the product of chromosomal DNA.

Example 2

Preparation of actinomycetal gene library

Actinomycetal chromosomal DNA (2 μg) was cleaved with 4 U of the restriction endonuclease Sal I (manufactured by TOYOBO Co.) at 37 °C for 2 hours in the presence of 50 mM Tris-HCl (pH 7.5), 100 mM NaCl, 10 mM $MgCl_2$, 1 mM DTT (dithiothreitol) and 10 μg/ml BSA (bovine serum albumin; BOEHRINGER-MANNHEIM Co.).

Also, 2 μg of the vector plasmid pUC 118 (ColE 1 ori, $Amp^r$, M 13 IG; manufactured by TAKARA SHUZO Co.) was cleaved with 4 U of Sal I at 37 °C for 16 hours under the same condition as above before treatment with phenol-chloroform and precipitation with ethanol. The linearized pUC 118 was reacted with the alkaline phosphatase derived from E. coli (manufactured by TOYOBO Co.) at 65 °C for 1 hour in the presence of 50 mM Tris-HCl (pH 8.0) and 1 mM $MgCl_2$ to remove the phosphate moiety at the 5′-terminus.

The pUC 118 (100 ng) and the actinomycetal chromosome (200 ng) thus treated were treated with 100 U of the T4 DNA ligase (manufactured by TAKARA SHUZO Co.) at 16 °C for 16 hours in the presence of 66 mM Tris-HCl (pH 7.6), 6.6 mM $MgCl_2$, 10 mM DTT (dithiothreitol) and 660 μM ATP (manufactured by BOEHRINGER-MANNHEIM). The product was transformed into E. coli DH 1 (ATCC 33849) ($F^-$, rec A 1, end A 1, gyr A96, thi-1, hsd R 17 ($\gamma_k^-$, $m_k^+$), Sup E 44, rel A 1, $\lambda^-$) [T. Maniatis et al., Molecular Cloning: Cold Spring Harbor (1982) 504 - 506] which had been modified into a competent cell by the method of K. Shigesada [SAIBO KOGAKU (1983) 2, 616 - 626] and cultured in an ampicillin (50 μg/ml) containing L plate agar culture medium [15 g/ℓ of Bacto Tripton (manufactured by DIFCO), 5 g/ℓ of yeast extract (manufactured by DIFCO), 5 g/ℓ of NaC1 and 15 g/ℓ of Bacto Agar (manufactured by DIFCO)] overnight to give about 100,000 ampicillin resistant colonies as the actinomycetal gene library.

Example 3

Preparation of radioactive oligonucleotide probe

Prior to the preparation of an oligonucleotide probe in a phospholipase D which had not been analyzed genetically, a phospholipase D obtained by the fermentation method was first purified completely, and the amino acid sequence in the N-terminal side of the phospholipase D was investigated. As a result, the sequence of 20 amino acids from the N-terminus was analyzed and determined as the amino acid sequence represented by the following A. Prior to the preparation of an oligonucleotide probe in a phospholipase D, the sequence of 60 bases from the 5′-terminal side of the gene was presumed as various kinds of oligonucleotides represented by the following B on the basis of the 20 amino acids from the N-terminal side thus determined. While innumerable sequences might be present as the oligonucleotide probe designed on the basis of the presumed sequences, several kinds of the sequences were designed in the present invention. As a result of several repeats of try and error, it was found that the oligonucleotide which consists of 50 bases and is represented by the following C is most useful in the present invention, and thus the oligonucleotide was used as a probe in the following operation.

(A) Amino acid sequence:

```
Ala Asp Gln Ala Pro Ala Phe Leu His Gly Val Ala Cys Gly Asp Pro Leu X Asp Ser
```

(B) Presumed DNA sequence:

```
GCN GAC CAA GCN CCN GCN TTC CTN CAC GGN GTN GCN TGC GGN GAC CCN CTN --- GAC TCN
      T   G               T TTA   T               T       T     TTA       T AGC
                              G                                   G           T
```

(C) Oligonucleotide:

```
GCG GAC CAG GCG CCG GCG TTC CTG CAC GGC GTC GCG TGC GGG GAC CCG CT
```

The oligonucleotide represented by C in the above was prepared with use of a DNA synthesizer (Beckman System 1 plus, manufactured by BECKMAN Co.) on the basis of the method by R.L. Letsinger, W.B. Lursford; J. Am. Chem. Soc., 98, 3655.

The oligonucleotide (200 ng) thus obtained was reacted with 8.5 U of T4 polynucleotide kinase (manufactured by TOYOBO Co.) at 37 °C for 30 minutes in the presence of a T4 polynucleotide kinase buffer (50 mM Tris-HCl, pH 8.0; 10 mM $MgCl_2$; 10 mM 2-mercaptoethanol) and 740 kBq of $^{32}$P-ATP

(manufactured by AMERCIAM JAPAN Co.) to give a radioactive oligonucleotide probe by incorporating the radioisotope $^{32}P$ into the oligonucleotide.

Example 4

Screening of phospholipase D gene containing clone

On the gene library thus obtained, that is the ampicillin resistant colonies on the plate agar culture medium was placed a nylon membrane filter (Biodine A, manufactured by PALL Co.), and a part of the colonies was moved onto the filter. The filter on which a part of the colonies had been moved was placed on another ampicillin (50 µg/ml) containing L agar plate culture medium, and the microorganism cell was cultured at 37°C for 16 hours. After culturing the cell, the filter was dipped into an alkali denaturating solution (0.5 N NaOH, 1.5 N NaCl) for 5 minutes, further dipped into 3 M sodium acetate buffer (pH 5.5) for 5 minutes and dried. The filter was heated at 80°C for 1 hour, and the plasmid DNA in the microorganism cell was immobilized on the filter.

The filter was further dipped into hybridization solution [NaCl (43.84 g/ℓ), trisodium citrate (22.1 g/ℓ), 50 mM trisodium phosphate (pH 6.5), sodium dodecylsulfate (1 g/ℓ), phycol (1 g/ℓ, manufactured by PHARMACIA Co.), polyvinylpyrrolidone (1 g/ℓ), BSA (1 g/ℓ, manufactured by BOEHRINGER-MANNHEIM Co.), salmon sperma DNA (250 mg/ℓ, manufactured by PHARMACIA Co.), formamide (0.4 l/ℓ)], and hybridization was conducted at 42°C for 1 hour. Then, the filter was dipped into a fresh hybridization solution, and the preliminarily prepared radioative oligonucleotide probe was added. Hybirdization was conducted at 42°C for 24 hours.

After hybridization, the filter was washed thrice with a washing solution [NaCl (4.38 g/ℓ), trisodium citrate (2.21 g/ℓ), sodium dodecylsulfate (1 g/ℓ)], and the filter was further dipped into the washing solution at 50°C to remove the excessive probe. The filter was air dried and placed on a film for X ray photography (New RXO-H, manufactured by FUJI PHOTO FILM Co.), and autoradiography was conducted at -70°C for 24 hours with shielding the light.

After the autoradiography was completed, the film was developed and colonies indicating positive signals were confirmed. The colonies were harvested as a transformant containing DNA which encoded the phospholipase D and deposited as E. coli DH1•pcPLD1 (FERM BP 2683).

In this connection, the optimum condition of hybridization varies depending on the shapes of the oligonucleotide. Therefore, in the present invention, it was found as result of repeated examinations with try and error that the oligonucleotide probe having the 50 bases which was represented by the above-described C was optimal under the aforementioned hybridization condition, and it was also possible to distinguish the positive signal of the transformant which was present alone among about 100,000 colonies and contained the gene of phospholipase D.

Example 5

Extraction of recombinant plasmid

The recombinant plasmid pcPLD1 containing the DNA which encoded the phospholipase D was extracted from E. coli DH1•pcPLD1 by the method of T. Maniatis et al., Molecular Cloning; Cold Spring Harbor, (1982) 86 - 94. The restriction enzyme map is shown in Fig. 4.

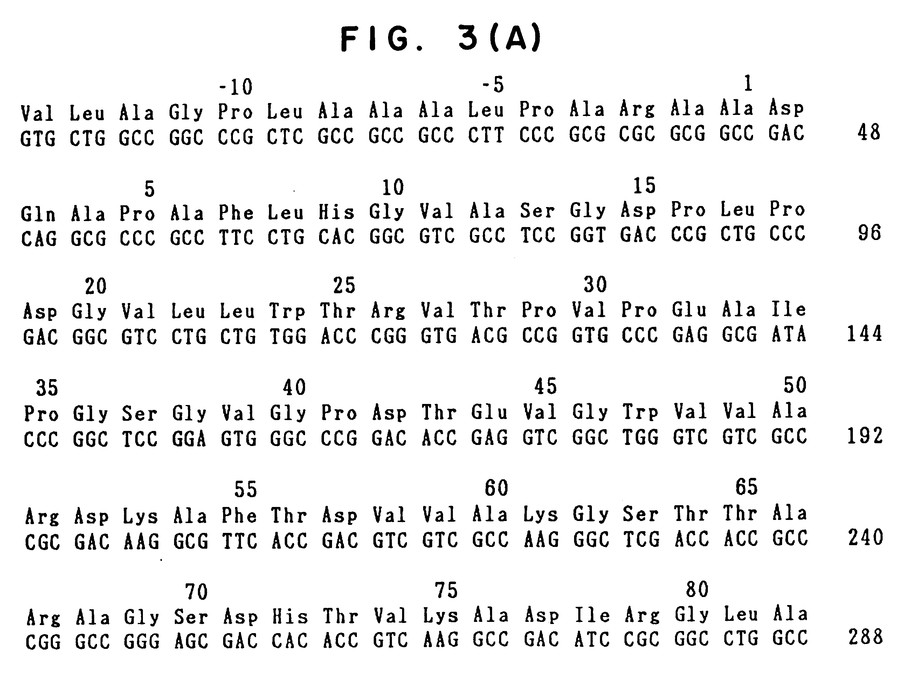

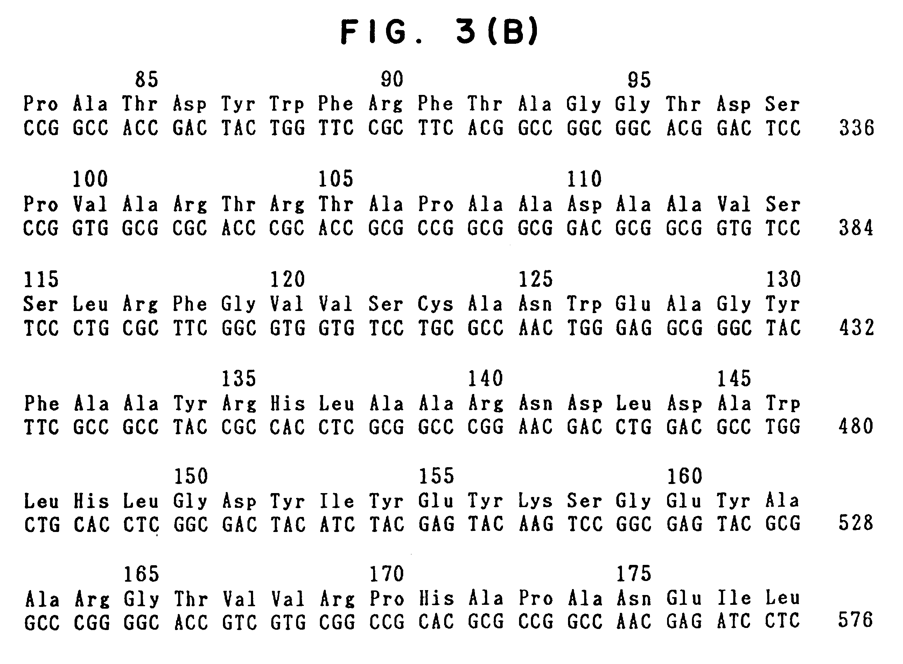

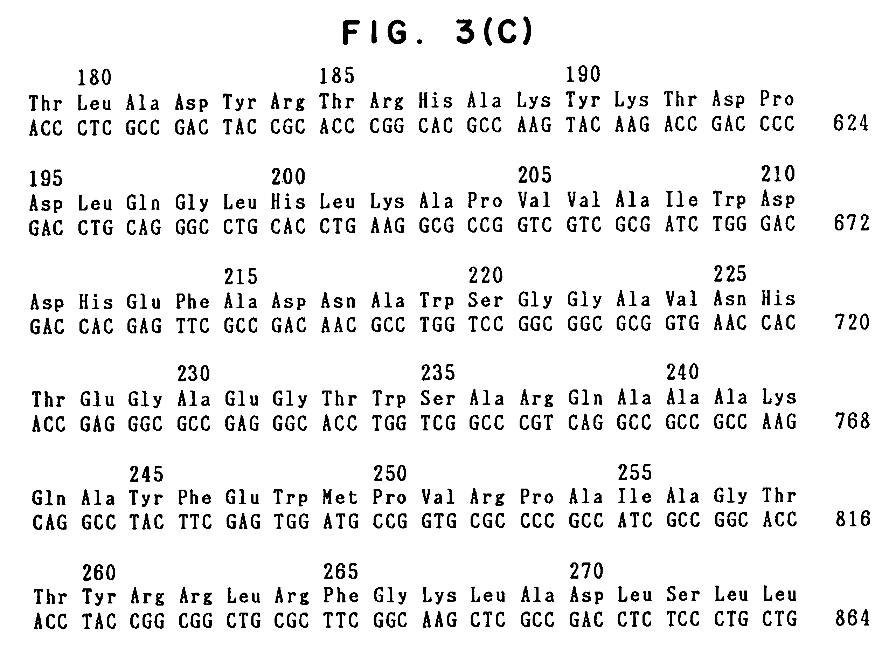

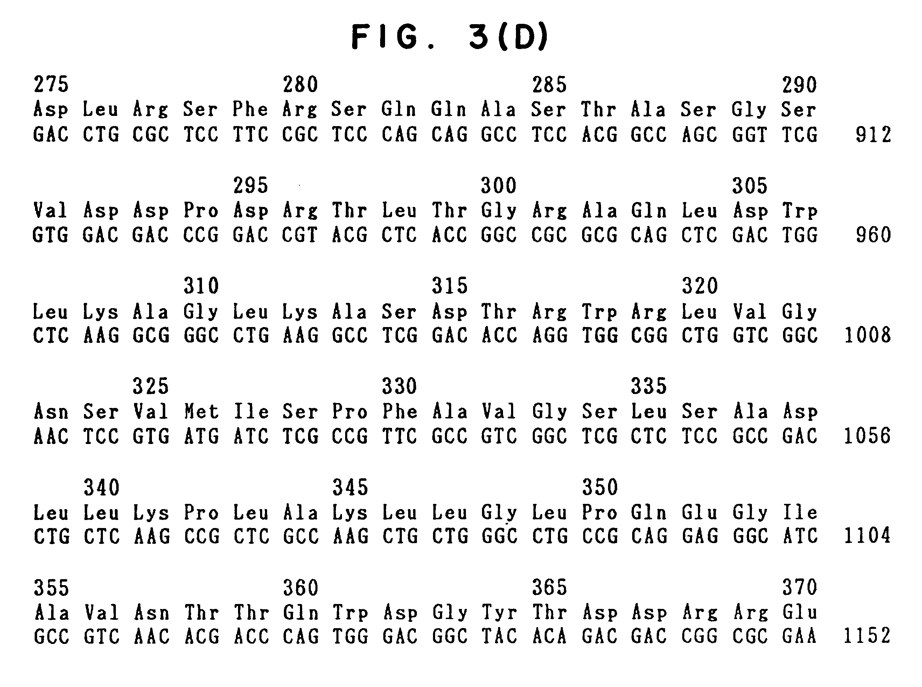

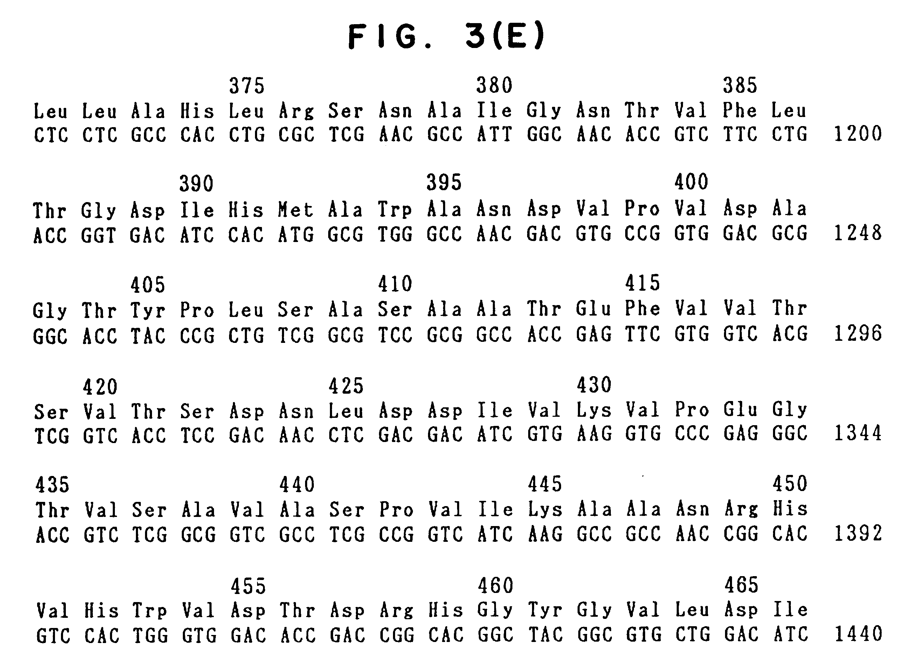

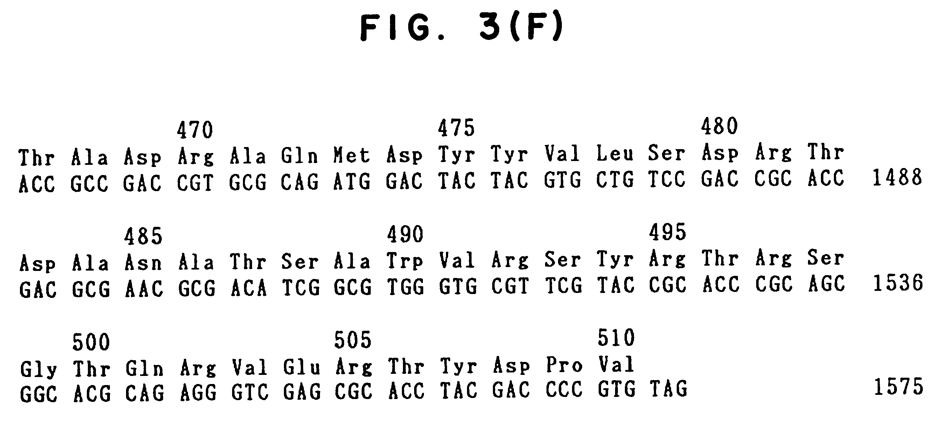

The base sequence of the DNA at the part derived from actinomycetal chromosome in the plasmid was determined by the dideoxy method [Science, 214, 1205 - 1210 (1981)], so that it was confirmed that the total DNA coding for the phospholipase D is contained. At the same time, the total base sequence was determined. The result is shown in Fig. 3.

Example 6

Culturing and preparation of cell extracts

E. coli DH1•pcPLD1 containing pcPLD1 was cultured in 20 ml of an ampicillin (50 µg/ml) containing LB culture medium [1.0% Bacto Tripton (manufactured by DIFCO Co.), 0.5% yeast extract (manufactured by DIFCO Co.), 1.0% NaCl] at 37°C for 18 hours, and the cells were collected by centrifugation (6,000 rpm, 10 minutes) and suspended in 100 µl of a 10 mM Tris-HCl (pH 8.0) buffer. The cells were crushed with a supersonic crusher and centrifuged at 14,000 rpm for 5 minutes, and the supernatant was collected as cell

extract.

Example 7

Confirmation of the phospholipase D activity of the cell extract

In order to confirm the expression of the PLD gene in E. coli DH1•pcPLD1, the phospholipase D activity in the cell extract was measured.

A 5 ml portion of a substrate solution (5 mM phosphatidylcholine, 3.8% chloroform, 1% Triton X-100, 40 mM Tris-HCl, pH 8.0, 10 mM $CaCl_2$) was sampled and maintained at a temperature of 37 °C for 5 minutes. The cell extract (50 μl) was added, and the mixture was stirred and reacted at 37 °C for 10 minutes. Next, 2.5 ml of Chromogen solution [0.6 mM 4-aminoantipyrine, phenol (80 μg/ml), 2.4 mM EDTA, 40 mM Tris-HCl (pH 8.0)] was added, and the mixture was stirred was reacted at 37 °C for 4 hours. The absorbance at 500 nm of the reaction solution was measured and taken as an index of the phospholipase D.

As the control, the extract of an untransformed E. coli DH 1 was treated in the same manner as above. The results are shown in Table 1.

It was confirmed that E. coli DH1 pcPLD1 expressed the phospholipase D activity.

Table 1

| Cell extract | Absorbance at 500 nm |
|---|---|
| E. coli DH1·pcPLD 1 | 0.055 |
| E. coli DH1 | 0.010 |

Example 8

Introduction of the phospholipase D gene into actinomycetes

The plasmid vector pcPLD 1 (2 μg) was cleaved with restriction endonucleases Mlu I (6 U) and Xho I (10 U) (manufactured by TOYOBO Co.), and the 3 kb DNA fragment containing the phospholipase D (PLD) gene is separated by electrophoresis on a 1% low melting agarose gel (manufactured by BETHESDA RESEARCH LABORATORIES Co.). The bothe ends of the fragment were changed into blunt ends by treating with 2.4 U of T4 DNA polymerase (manufactured by TOYOBO Co.) in the presence of 67 mM Tris-HCl (pH 8.8), 6.7 mM $MgCl_2$, 16.6 mM $(NH_4)_2SO_4$, 10 mM 2-mercaptoethanol, 6.7 μM EDTA, 50 μM dNTP (manufactured by BOEHRINGER-MANNHEIM) and 167 μg/ml BSA (manufactured by BOEHRINGER-MANNHEIM). The actinomycetal vector plasmid pSEV 1 (2 μg) [S. Horinovchi, M. Nishiyama, A. Nakamura, T. Beppu, Mol. Gen. Genet., (1987) 210: 468 - 475 (FERM BP 2684)] was cleaved with 2 U of a restriction endonuclease Sac I (manufactured by TOYOBO Co.). The both ends of the fragment were changed into blunt ends by the treatment in the same manner as above, and the fragment was treated with 6 U of a bacterial alkaline phosphatase (manufactured by TOYOBO Co.) to remove the phosphate moieties at both ends. The PLD gene containing fragment (200 ng) thus treated and the plasmid pSEV1 (100 ng) were ligated with 100 U of T4 NDA ligase (manufactured by TAKARA SHUZO Co.) in the presence of 6.6 mM Tris-HCl (pH 7.6), 6.6 mM $MgCl_2$, 10 mM DTT and 660 μM ATP (manufactured by BOEHRINGER-MANNHEIM) at 16 °C for 16 hours. The ligated product was transformed into S. lividans TK-24 (FERM BP 2685) which had been made into a competent cell by the method of D.A. Hopwood et al. (Genetic Manipulation of Streptomyces. A Laboratory Manual. the John Innes Foundation, John Innes Institute, 103 - 122), spread on a Triptic Soy plate agar medium (3% Triptic Soy, 2% Bacto Agar (manufactured by DIFCO Co., respectively) and cultured at 28 °C for 1 day. Then, the Triptic Soy plate agar was covered with a Nutrient Broth agar medium containing thiostrepton (100 μg/mg) [1.8% Nutrient Broth (manufactured by

TANABE PHARMACEUTICAL Co.), 11.3% sucrose, 0.5% agar (manufactured by WAKO PURE CHEMICAL INDUSTRIES, LTD)], and culture was conducted at 28 °C for 1 day to give a thiostrepton resistant colony, which was regarded as an actinomycetes strain containing the plasmid pSEV-PLD as the PLD gene containing vector (see Fig. 5). The transformant thus obtained was deposited as Streptomyces lividans TK-24•pSEV1-PLD (FERM BP 2686).

Example 9

Culture of Streptomyces lividans TK-24•pSEV1-PLD and the expression of the activity

Streptomyces lividans TK-24•pSEV1-PLD which was the transformant of the actinomycetes thus obtained was cultured in a 3% Triptic Soy (manufactured by DIFCO Co.) culture medium at 28 °C for 3 days. The culture was centrifuged at 14,000 rpm for 2 minutes, and the supernatant was separated. The phospholipase D activity of the culture supernatant was measured in the same manner as in Example 7. The phospholipase D activity of the culture supernatant of an untransformed Streptomyces lividans TK-24 was also measured. The results are shown in Table 2. It was confirmed that the phospholipase D activity was expressed by the introduction of the vector pSEV-PLD.

Table 2

| Culture Supernatant | Phospholipase D activity (U/ml) |
|---|---|
| Transformant | 1.36 |
| Untransformed | 0.064 |

According to the present invention, a novel phospholipase D gene of which the total DNA sequence had been defined was separated from a chromosomal DNA library derived from a phospholipase D producing strain with use of an oligonucleotide synthesized on the basis of the N-terminal amino acid sequence of phospolipase D. The use of the phospholipase D gene made it possible to produce and culture phospholipase D in any host-vector systems. Furthermore, a variety of protein engineerings such as the improvement of the heat resistance or the change of the substrate specificity of the phospholipase D were also made possible by the isolation of the phospholipase D gene.

SEQUENCE LISTING

SEQ ID NO: 1
SEQUENCE TYPE: Nucleotide with corresponding protein
SEQUENCE LENGTH: 1530 base pairs

STRANDEDNESS: UNKNOWN
TOPOLOGY: Linear
MOLECULE TYPE: genomic DNA

ORIGINAL SOURCE
ORGANISM: microorganism
IMMEDIATE EXPERIMENTAL SOURCE:
Streptomyces chromofuscus A- 0848

PROPERTIES: phospholipase D.gene

## Claims

1. A DNA, wherein the DNA sequence from the 5'-terminus is represented by Fig.2.

2. A vector characterized in that it maintains a DNA sequence from the 5'-terminus represented by Fig.2.

3. A vector according to claim 2, wherein said vector is the plasmid pcPLD1 contained in Escherichia coli DH1.pcPLD1 (FERM BP 2683). or the plasmid pSEV1-PLD contained in Streptomyces lividans TK24.pSEV1-PLD(FERM BP 2686).

4. A transformant characterized in that a host is a microorganism belonging to Escherichia genus, Streptomyces genus, Saccharomyces genus or Bacillus genus, which maintains a DNA exogenous to the host, wherein the DNA sequence from the 5'-terminus is represented by Fig. 2.

5. A transformant according to claim 4, wherein the microorganism belonging to Escherichia genus is a microorganism belonging to Escherichia coli.

6. A transformant according to claim 5, wherein the transformant is Escherichia coli DH1-pcPLD1 (FERM BP 2683).

7. A transformant according to claim 4, wherein the microorganism belonging to Streptomyces genus is a microorganism belonging to Streptomyces lividans.

8. A transformant according to claim 7, wherein the transformant is Streptomyces lividans TK24•pSEV1-PLD (FERM BP 2686).

9. A process for producing phospholipase D. characterized in that a host is a microorganism belonging to Escherichia genus, Streptomyces genus, Saccharomyces genus or Bacillus genus, wherein a transformant maintaining the DNA represented by Fig. 2 is cultured to express the genetic informations of said DNA and phospholipase D is harvested from said culture.

**Patentansprüche**

1. Eine DNA, für die die DNA-Sequenz vom 5'-Terminus durch Figur 2 wiedergegeben ist.

2. Ein Vektor, dadurch **gekennzeichnet**, daß er eine DNA-Sequenz vom 5'-Terminus gemäß Figur 2 umfaßt.

3. Ein Vektor nach Anspruch 2, wobei es sich bei dem Vektor um das Plasmid pcPLD1 in *Escherichia coli* DH1.pcPLD1 (FERM BP 2683) oder das Plasmid pSEV1-PLD in *Streptomyces lividans* TK24.pSEV1-PLD (FERM BP 2686) handelt.

4. Ein Transformant, gekennzeichnet durch einen Wirt, bei dem es sich um einen Mikroorganismus handelt, der zur Gattung *Escherichia*, zur Gattung *Streptomyces*, zur Gattung *Saccharomyces* oder zur Gattung *Bacillus* gehört, enthaltend eine für den Wirt exogene DNA, wobei die DNA-Sequenz vom 5'-Terminus durch die Figur 2 wiedergegeben ist.

5. Ein Transformant nach Anspruch 4, wobei der Mikroorganismus der Gattung *Escherichia* ein Mikroorganismus ist, der zu *Escherichia coli* gehört.

6. Ein Transformant nach Anspruch 5, wobei der Transformant *Escherichia coli* DH1-pcPLD1 (FERM BP 2683) ist.

7. Ein Transformant nach Anspruch 4, wobei der Mikroorganismus der Gattung *Streptomyces* ein Mikroorganismus ist, der zu *Streptomyces lividans* gehört.

8. Ein Transformant nach Anspruch 7, wobei der Transformant *Streptomyces lividans* TK24.pSEV1-PLD (FERM BP 2686) ist.

9. Verfahren zur Herstellung von Phospholipase D, gekennzeichnet durch einen Wirt, der ein Mikroorganismus der Gattung *Escherichia*, der Gattung *Streptomyces*, der Gattung *Saccharomyces* oder der Gattung *Bacillus* ist, wobei ein Transformant, der eine DNA gemäß Figur 2 umfaßt, kultiviert wird und

die genetische Information der DNA exprimiert wird und Phospholipase D von der Kultur geerntet wird.

## Revendications

1. ADN, dans lequel la séquence d'ADN depuis l'extrémité 5' est représentée par la figure 2.

2. Vecteur caractérisé en ce qu'il conserve une séquence d'ADN depuis l'extrémité 5' représentée à la figure 2.

3. Vecteur selon la revendication 2, qui est le plasmide pcPLD1 contenu dans l'Escherichia Coli DH1.pcPLD1 (FERM BP 2683) ou le plasmide pSEV1-PLD contenu dans le Streptomyces lividans TK24.pSEV1-PLD (FERM BP 2686).

4. Transformant caractérisé en ce qu'un hôte est un microorganisme appartenant au genre Escherichia, au genre Streptomyces, au genre Saccharomyces ou au genre Bacillus, qui conserve un ADN exogène à l'hôte, dans lequel la séquence d'ADN à partir de l'extrémité 5' est représentée par le figure 2.

5. Transformant selon la revendication 4, dans lequel le microorganisme appartenant au genre Escherichia est un microorganisme appartenant à l'Escherichia Coli.

6. Transformant selon la revendication 5, qui est l'Escherichia Coli DH1-pc PLD1 (FERM BP 2683).

7. Transformant selon la revendication 4, dans lequel le microorganisme appartenant au genre Streptomyces est un microorganisme appartenant au Streptomyces lividans.

8. Transformant selon la revendication 7, qui est le Streptomyces lividans TK24•PSEV1-PLD (FERM BP 2686).

9. Procédé de production de phospholipase D. caractérisé en ce que un hôte est un microorganisme appartenant au genre Escherichia, au genre Streptomyces, au genre Saccharomyces ou au genre Bacillus, qui consiste à mettre en culture un transformant conservant l'ADN représenté à la figure 2 afin d'exprimer les informations génétiques dudit ADN et récolter dans cette culture la phospholipase D.

# FIG. 1(A)

1 5 10 15
Ala Asp Gln Ala Pro Ala Phe Leu His Gly Val Ala Ser Gly Asp Pro

20 25 30
Leu Pro Asp Gly Val Leu Leu Trp Thr Arg Val Thr Pro Val Pro Glu

35 40 45
Ala Ile Pro Gly Ser Gly Val Gly Pro Asp Thr Glu Val Gly Trp Val

50 55 60
Val Ala Arg Asp Lys Ala Phe Thr Asp Val Val Ala Lys Gly Ser Thr

65 70 75 80
Thr Ala Arg Ala Gly Ser Asp His Thr Val Lys Ala Asp Ile Arg Gly

85 90 95
Leu Ala Pro Ala Thr Asp Tyr Trp Phe Arg Phe Thr Ala Gly Gly Thr

100 105 110
Asp Ser Pro Val Ala Arg Thr Arg Thr Ala Pro Ala Ala Asp Ala Ala

115 120 125
Val Ser Ser Leu Arg Phe Gly Val Val Ser Cys Ala Asn Trp Glu Ala

130 135 140
Gly Tyr Phe Ala Ala Tyr Arg His Leu Ala Ala Arg Asn Asp Leu Asp

# FIG. 1(B)

```
145                 150                 155                 160
Ala Trp Leu His Leu Gly Asp Tyr Ile Tyr Glu Tyr Lys Ser Gly Glu

                165                 170                 175
Tyr Ala Ala Arg Gly Thr Val Val Arg Pro His Ala Pro Ala Asn Glu

            180                 185                 190
Ile Leu Thr Leu Ala Asp Tyr Arg Thr Arg His Ala Lys Tyr Lys Thr

        195                 200                 205
Asp Pro Asp Leu Gln Gly Leu His Leu Lys Ala Pro Val Val Ala Ile

    210                 215                 220
Trp Asp Asp His Glu Phe Ala Asp Asn Ala Trp Ser Gly Gly Ala Val

225                 230                 235                 240
Asn His Thr Glu Gly Ala Glu Gly Thr Trp Ser Ala Arg Gln Ala Ala

                245                 250                 255
Ala Lys Gln Ala Tyr Phe Glu Trp Met Pro Val Arg Pro Ala Ile Ala

            260                 265                 270
Gly Thr Thr Tyr Arg Arg Leu Arg Phe Gly Lys Leu Ala Asp Leu Ser

        275                 280                 285
Leu Leu Asp Leu Arg Ser Phe Arg Ser Gln Gln Ala Ser Thr Ala Ser
```

# FIG. 1(C)

```
    290                     295                     300
Gly Ser Val Asp Asp Pro Asp Arg Thr Leu Thr Gly Arg Ala Gln Leu

305                     310                     315                     320
Asp Trp Leu Lys Ala Gly Leu Lys Ala Ser Asp Thr Arg Trp Arg Leu

                325                     330                     335
Val Gly Asn Ser Val Met Ile Ser Pro Phe Ala Val Gly Ser Leu Ser

            340                     345                     350
Ala Asp Leu Leu Lys Pro Leu Ala Lys Leu Leu Gly Leu Pro Gln Glu

        355                     360                     365
Gly Ile Ala Val Asn Thr Thr Gln Trp Asp Gly Tyr Thr Asp Asp Arg

    370                     375                     380
Arg Glu Leu Leu Ala His Leu Arg Ser Asn Ala Ile Gly Asn Thr Val

385                     390                     395                     400
Phe Leu Thr Gly Asp Ile His Met Ala Trp Ala Asn Asp Val Pro Val

                405                     410                     415
Asp Ala Gly Thr Tyr Pro Leu Ser Ala Ser Ala Ala Thr Glu Phe Val

            420                     425                     430
Val Thr Ser Val Thr Ser Asp Asn Leu Asp Asp Ile Val Lys Val Pro
```

# FIG. 1(D)

```
        435                     440                     445
Glu Gly Thr Val Ser Ala Val Ala Ser Pro Val Ile Lys Ala Ala Asn

    450                 455                 460
Arg His Val His Trp Val Asp Thr Asp Arg His Gly Tyr Gly Val Leu

465                 470                 475                 480
Asp Ile Thr Ala Asp Arg Ala Gln Met Asp Tyr Tyr Val Leu Ser Asp

                485                 490                 495
Arg Thr Asp Ala Asn Ala Thr Ser Ala Trp Val Arg Ser Tyr Arg Thr

            500                 505                 510
Arg Ser Gly Thr Gln Arg Val Glu Arg Thr Tyr Asp Pro Val
```

# FIG. 2(A)

```
GCCGACCAGG CGCCCGCCTT CCTGCACGGC GTCGCCTCCG GTGACCCGCT GCCCGACGGC    60
GTCCTGCTGT GGACCCGGGT GACGCCGGTG CCCGAGGCGA TACCCGGCTC CGGAGTGGGC   120
CCGGACACCG AGGTCGGCTG GGTCGTCGCC CGCGACAAGG CGTTCACCGA CGTCGTCGCC   180
AAGGGCTCGA CCACCGCCCG GGCCGGGAGC GACCACACCG TCAAGGCCGA CATCCGCGGC   240
CTGGCCCCGG CCACCGACTA CTGGTTCCGC TTCACGGCCG GCGGCACGGA CTCCCCGGTG   300
GCGCGCACCC GCACCGCGCC GGCGGCGGAC GCGGCGGTGT CCTCCCTGCG CTTCGGCGTG   360
GTGTCCTGCG CCAACTGGGA GGCGGGCTAC TTCGCCGCCT ACCGCCACCT CGCGGCCCGG   420
AACGACCTGG ACGCCTGGCT GCACCTCGGC GACTACATCT ACGAGTACAA GTCCGGCGAG   480
TACGCGGCCC GGGGCACCGT CGTGCGGCCG CACGCGCCGG CCAACGAGAT CCTCACCCTC   540
GCCGACTACC GCACCCGGCA CGCCAAGTAC AAGACCGACC CCGACCTGCA GGGCCTGCAC   600
CTGAAGGCGC CGGTCGTCGC GATCTGGGAC GACCACGAGT TCGCCGACAA CGCCTGGTCC   660
GGCGGCGCGG TGAACCACAC CGAGGGCGCC GAGGGCACCT GGTCGGCCCG TCAGGCCGCC   720
GCCAAGCAGG CCTACTTCGA GTGGATGCCG GTGCGCCCCG CCATCGCCGG CACCACCTAC   780
```

# FIG. 2(B)

```
CGGCGGCTGC GCTTCGGCAA GCTCGCCGAC CTCTCCCTGC TGGACCTGCG CTCCTTCCGC  840
TCCCAGCAGG CCTCCACGGC CAGCGGTTCG GTGGACGACC CGGACCGTAC GCTCACCGGC  900
CGCGCGCAGC TCGACTGGCT CAAGGCGGGC CTGAAGGCCT CGGACACCAG GTGGCGGCTG  960
GTCGGCAACT CCGTGATGAT CTCGCCGTTC GCCGTCGGCT CGCTCTCCGC CGACCTGCTC 1020
AAGCCGCTCG CCAAGCTGCT GGGCCTGCCG CAGGAGGGCA TCGCCGTCAA CACGACCCAG 1080
TGGGACGGCT ACACAGACGA CCGGCGCGAA CTCCTCGCCC ACCTGCGCTC GAACGCCATT 1140
GGCAACACCG TCTTCCTGAC CGGTGACATC CACATGGCGT GGGCCAACGA CGTGCCGGTG 1200
GACGCGGGCA CCTACCCGCT GTCGGCGTCC GCGGCCACCG AGTTCGTGGT CACGTCGGTC 1260
ACCTCCGACA ACCTCGACGA CATCGTGAAG GTGCCCGAGG GCACCGTCTC GGCGGTCGCC 1320
TCGCCGGTCA TCAAGGCCGC CAACCGGCAC GTCCACTGGG TGGACACCGA CCGGCACGGC 1380
TACGGCGTGC TGGACATCAC CGCCGACCGT GCGCAGATGG ACTACTACGT GCTGTCCGAC 1440
CGCACCGACG CGAACGCGAC ATCGGCGTGG GTGCGTTCGT ACCGCACCCG CAGCGGCACG 1500
CAGAGGGTCG AGCGCACCTA CGACCCCGTG                                  1530
```

# FIG. 3(A)

```
                -10                     -5                      1
Val Leu Ala Gly Pro Leu Ala Ala Ala Leu Pro Ala Arg Ala Ala Asp
GTG CTG GCC GGC CCG CTC GCC GCC GCC CTT CCC GCG CGC GCG GCC GAC     48

            5                   10                  15
Gln Ala Pro Ala Phe Leu His Gly Val Ala Ser Gly Asp Pro Leu Pro
CAG GCG CCC GCC TTC CTG CAC GGC GTC GCC TCC GGT GAC CCG CTG CCC     96

    20                  25                  30
Asp Gly Val Leu Leu Trp Thr Arg Val Thr Pro Val Pro Glu Ala Ile
GAC GGC GTC CTG CTG TGG ACC CGG GTG ACG CCG GTG CCC GAG GCG ATA    144

35                  40                  45                  50
Pro Gly Ser Gly Val Gly Pro Asp Thr Glu Val Gly Trp Val Val Ala
CCC GGC TCC GGA GTG GGC CCG GAC ACC GAG GTC GGC TGG GTC GTC GCC    192

                55                  60                  65
Arg Asp Lys Ala Phe Thr Asp Val Val Ala Lys Gly Ser Thr Thr Ala
CGC GAC AAG GCG TTC ACC GAC GTC GTC GCC AAG GGC TCG ACC ACC GCC    240

            70                  75                  80
Arg Ala Gly Ser Asp His Thr Val Lys Ala Asp Ile Arg Gly Leu Ala
CGG GCC GGG AGC GAC CAC ACC GTC AAG GCC GAC ATC CGC GGC CTG GCC    288
```

# FIG. 3(B)

```
        85                      90                      95
Pro Ala Thr Asp Tyr Trp Phe Arg Phe Thr Ala Gly Gly Thr Asp Ser
CCG GCC ACC GAC TAC TGG TTC CGC TTC ACG GCC GGC GGC ACG GAC TCC   336

    100                     105                     110
Pro Val Ala Arg Thr Arg Thr Ala Pro Ala Ala Asp Ala Ala Val Ser
CCG GTG GCG CGC ACC CGC ACC GCG CCG GCG GCG GAC GCG GCG GTG TCC   384

115                     120                     125                     130
Ser Leu Arg Phe Gly Val Val Ser Cys Ala Asn Trp Glu Ala Gly Tyr
TCC CTG CGC TTC GGC GTG GTG TCC TGC GCC AAC TGG GAG GCG GGC TAC   432

                135                     140                     145
Phe Ala Ala Tyr Arg His Leu Ala Ala Arg Asn Asp Leu Asp Ala Trp
TTC GCC GCC TAC CGC CAC CTC GCG GCC CGG AAC GAC CTG GAC GCC TGG   480

            150                     155                     160
Leu His Leu Gly Asp Tyr Ile Tyr Glu Tyr Lys Ser Gly Glu Tyr Ala
CTG CAC CTC GGC GAC TAC ATC TAC GAG TAC AAG TCC GGC GAG TAC GCG   528

        165                     170                     175
Ala Arg Gly Thr Val Val Arg Pro His Ala Pro Ala Asn Glu Ile Leu
GCC CGG GGC ACC GTC GTG CGG CCG CAC GCG CCG GCC AAC GAG ATC CTC   576
```

# FIG. 3(C)

```
    180                 185                 190
Thr Leu Ala Asp Tyr Arg Thr Arg His Ala Lys Tyr Lys Thr Asp Pro
ACC CTC GCC GAC TAC CGC ACC CGG CAC GCC AAG TAC AAG ACC GAC CCC   624

195                 200                 205                 210
Asp Leu Gln Gly Leu His Leu Lys Ala Pro Val Val Ala Ile Trp Asp
GAC CTG CAG GGC CTG CAC CTG AAG GCG CCG GTC GTC GCG ATC TGG GAC   672

                215                 220                 225
Asp His Glu Phe Ala Asp Asn Ala Trp Ser Gly Gly Ala Val Asn His
GAC CAC GAG TTC GCC GAC AAC GCC TGG TCC GGC GGC GCG GTG AAC CAC   720

            230                 235                 240
Thr Glu Gly Ala Glu Gly Thr Trp Ser Ala Arg Gln Ala Ala Ala Lys
ACC GAG GGC GCC GAG GGC ACC TGG TCG GCC CGT CAG GCC GCC GCC AAG   768

        245                 250                 255
Gln Ala Tyr Phe Glu Trp Met Pro Val Arg Pro Ala Ile Ala Gly Thr
CAG GCC TAC TTC GAG TGG ATG CCG GTG CGC CCC GCC ATC GCC GGC ACC   816

    260                 265                 270
Thr Tyr Arg Arg Leu Arg Phe Gly Lys Leu Ala Asp Leu Ser Leu Leu
ACC TAC CGG CGG CTG CGC TTC GGC AAG CTC GCC GAC CTC TCC CTG CTG   864
```

# FIG. 3(D)

```
275                     280                     285                     290
Asp Leu Arg Ser Phe Arg Ser Gln Gln Ala Ser Thr Ala Ser Gly Ser
GAC CTG CGC TCC TTC CGC TCC CAG CAG GCC TCC ACG GCC AGC GGT TCG    912

                    295                     300                     305
Val Asp Asp Pro Asp Arg Thr Leu Thr Gly Arg Ala Gln Leu Asp Trp
GTG GAC GAC CCG GAC CGT ACG CTC ACC GGC CGC GCG CAG CTC GAC TGG    960

                310                     315                     320
Leu Lys Ala Gly Leu Lys Ala Ser Asp Thr Arg Trp Arg Leu Val Gly
CTC AAG GCG GGC CTG AAG GCC TCG GAC ACC AGG TGG CGG CTG GTC GGC   1008

            325                     330                     335
Asn Ser Val Met Ile Ser Pro Phe Ala Val Gly Ser Leu Ser Ala Asp
AAC TCC GTG ATG ATC TCG CCG TTC GCC GTC GGC TCG CTC TCC GCC GAC   1056

        340                     345                     350
Leu Leu Lys Pro Leu Ala Lys Leu Leu Gly Leu Pro Gln Glu Gly Ile
CTG CTC AAG CCG CTC GCC AAG CTG CTG GGC CTG CCG CAG GAG GGC ATC   1104

355                     360                     365                     370
Ala Val Asn Thr Thr Gln Trp Asp Gly Tyr Thr Asp Asp Arg Arg Glu
GCC GTC AAC ACG ACC CAG TGG GAC GGC TAC ACA GAC GAC CGG CGC GAA   1152
```

# FIG. 3(E)

```
                375                     380                     385
Leu Leu Ala His Leu Arg Ser Asn Ala Ile Gly Asn Thr Val Phe Leu
CTC CTC GCC CAC CTG CGC TCG AAC GCC ATT GGC AAC ACC GTC TTC CTG  1200

            390                     395                     400
Thr Gly Asp Ile His Met Ala Trp Ala Asn Asp Val Pro Val Asp Ala
ACC GGT GAC ATC CAC ATG GCG TGG GCC AAC GAC GTG CCG GTG GAC GCG  1248

        405                     410                     415
Gly Thr Tyr Pro Leu Ser Ala Ser Ala Ala Thr Glu Phe Val Val Thr
GGC ACC TAC CCG CTG TCG GCG TCC GCG GCC ACC GAG TTC GTG GTC ACG  1296

    420                     425                     430
Ser Val Thr Ser Asp Asn Leu Asp Asp Ile Val Lys Val Pro Glu Gly
TCG GTC ACC TCC GAC AAC CTC GAC GAC ATC GTG AAG GTG CCC GAG GGC  1344

435                     440                     445                     450
Thr Val Ser Ala Val Ala Ser Pro Val Ile Lys Ala Ala Asn Arg His
ACC GTC TCG GCG GTC GCC TCG CCG GTC ATC AAG GCC GCC AAC CGG CAC  1392

                455                     460                     465
Val His Trp Val Asp Thr Asp Arg His Gly Tyr Gly Val Leu Asp Ile
GTC CAC TGG GTG GAC ACC GAC CGG CAC GGC TAC GGC GTG CTG GAC ATC  1440
```

# FIG. 3(F)

```
                    470                     475                     480
    Thr Ala Asp Arg Ala Gln Met Asp Tyr Tyr Val Leu Ser Asp Arg Thr
    ACC GCC GAC CGT GCG CAG ATG GAC TAC TAC GTG CTG TCC GAC CGC ACC  1488

                485                     490                     495
    Asp Ala Asn Ala Thr Ser Ala Trp Val Arg Ser Tyr Arg Thr Arg Ser
    GAC GCG AAC GCG ACA TCG GCG TGG GTG CGT TCG TAC CGC ACC CGC AGC  1536

            500                     505                     510
    Gly Thr Gln Arg Val Glu Arg Thr Tyr Asp Pro Val
    GGC ACG CAG AGG GTC GAG CGC ACC TAC GAC CCC GTG TAG              1575
```

FIG. 4

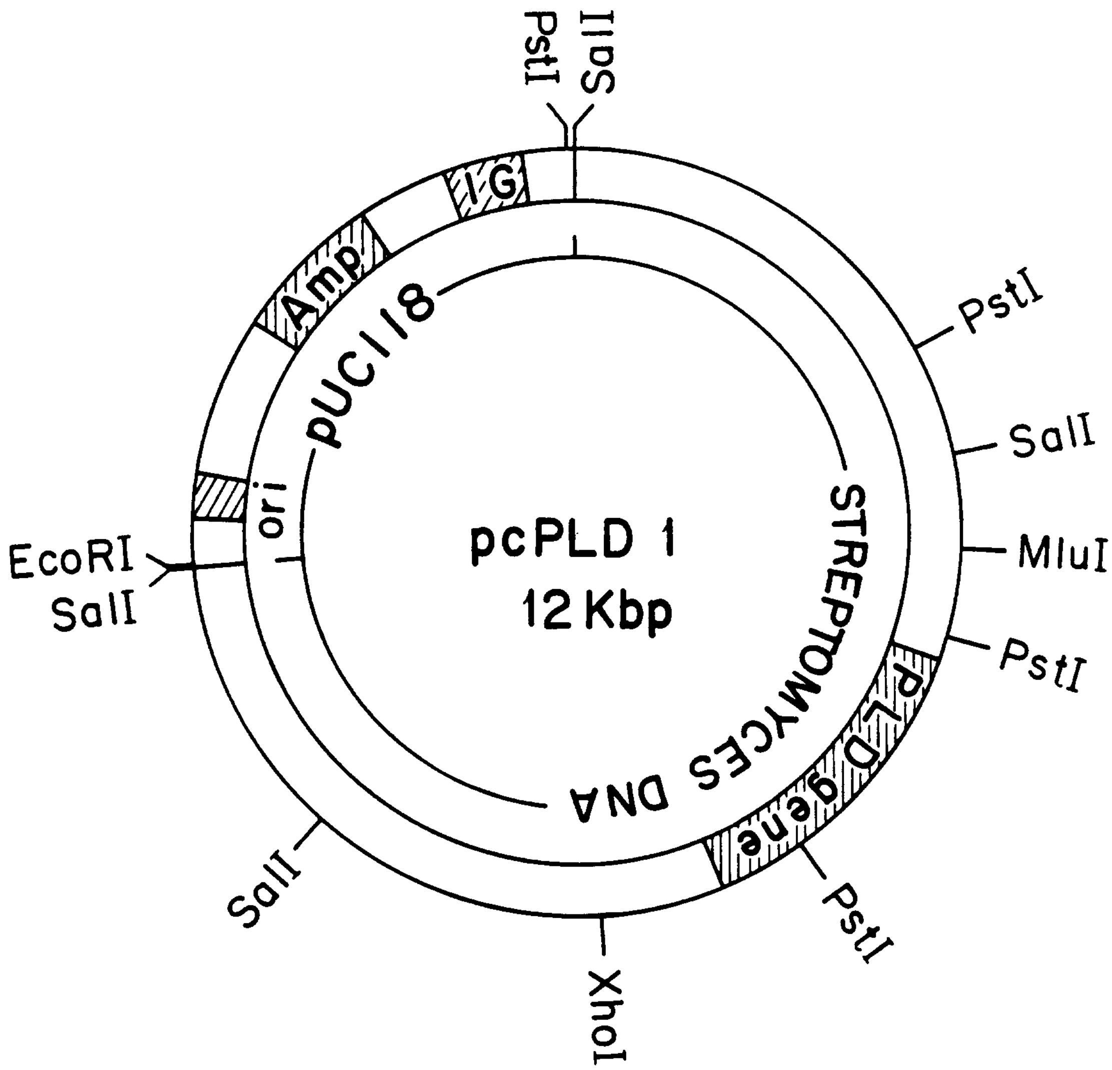
PstI
SalI
IG
Amp
pUC118
ori
EcoRI
SalI
PstI
SalI
MluI
PstI
STREPTOMYCES DNA
PLD gene
PstI
XhoI
SalI
pcPLD 1
12 Kbp

FIG. 5

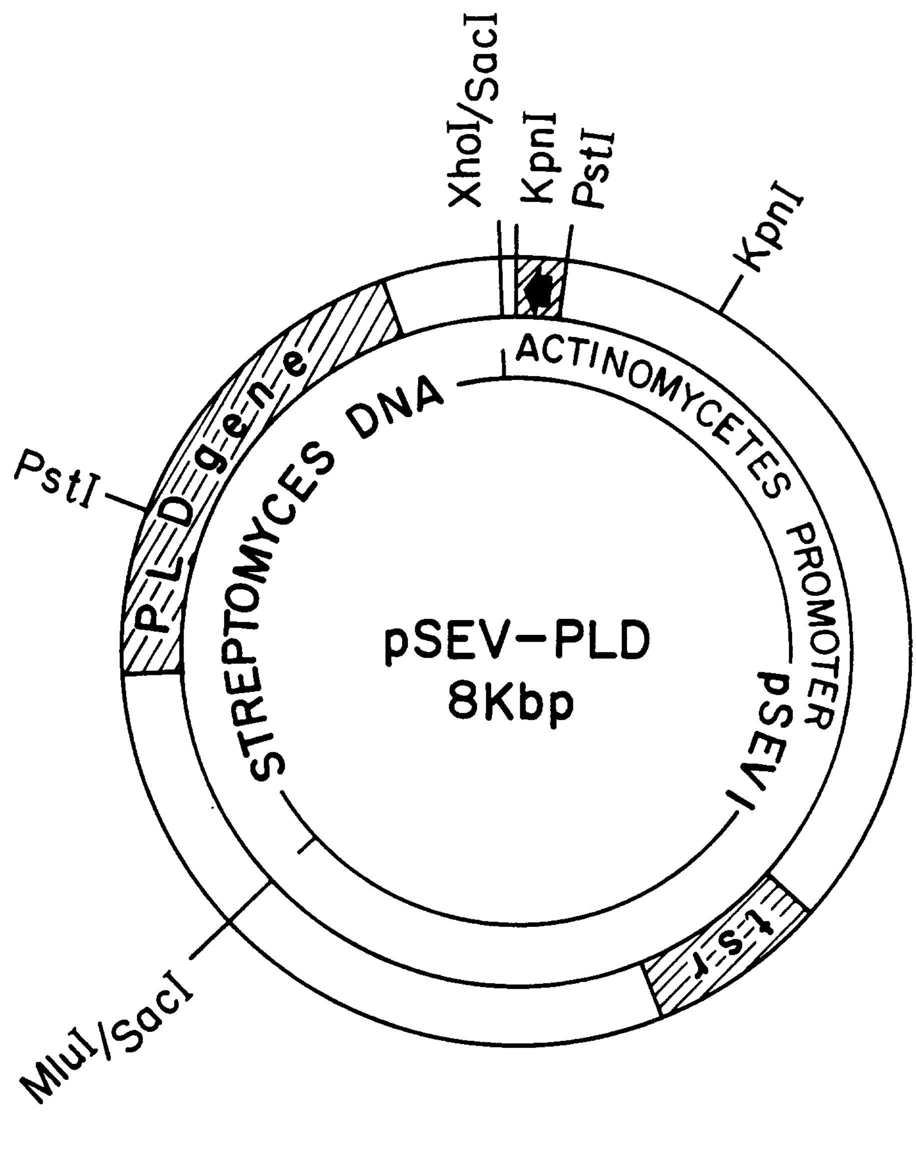
XhoI/SacI
KpnI
PstI
KpnI
PstI
P-L-D gene
STREPTOMYCES DNA
ACTINOMYCETES PROMOTER
pSEV-PLD
8Kbp
pSEV1
tsr
MluI/SacI

FIG. 6

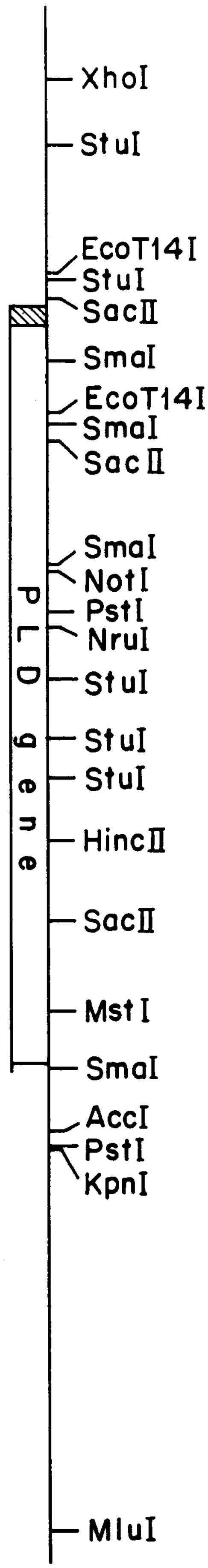
XhoI
StuI
EcoT14I
StuI
SacII
SmaI
EcoT14I
SmaI
SacII
SmaI
NotI
PstI
NruI
StuI
StuI
StuI
HincII
SacII
MstI
SmaI
AccI
PstI
KpnI
MluI
PLD gene

FIG. 7

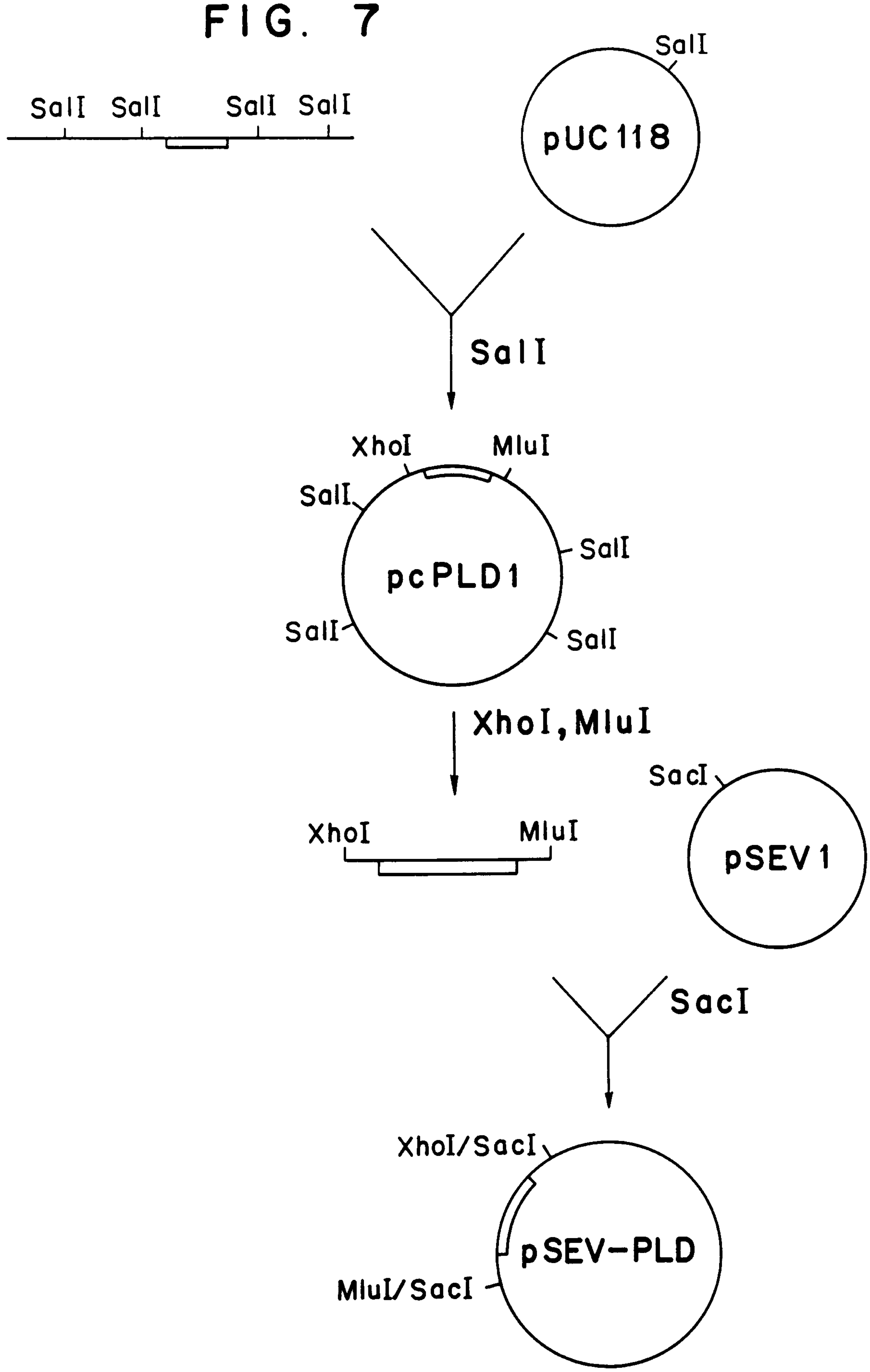
SalI
SalI
SalI
SalI
SalI
pUC118
SalI
XhoI
MluI
SalI
SalI
pcPLD1
SalI
SalI
XhoI, MluI
SacI
XhoI
MluI
pSEV1
SacI
XhoI/SacI
pSEV-PLD
MluI/SacI